# EUROPEAN PATENT SPECIFICATION

(11) **EP 3 177 155 B1**
(45) Date of publication and mention of the grant of the patent: **25.09.2019**
(21) Application number: 15750353.3
(22) Date of filing: 06.08.2015
(51) Int. Cl.: A23L 2/39, A23C 9/152, A61K 31/047, A61K 35/745, A61K 35/747, A61P 3/10

(54) **MYO-INOSITOL AND ONE OR MORE PROBIOTIC AND USE THEREOF**
MYO-INOSITOL UND EINES ODER MEHRERE PROBIOTIKA UND VERWENDUNG DAVON
MYO-INOSITOL ET UN OU PLUSIEURS PROBIOTIQUES ET UTILISATION DE CEUX-CI

(30) Priority: 08.08.2014 EP 14180399; 08.08.2014 EP 14180403; 05.06.2015 EP 15170915
(43) Date of publication of application: 14.06.2017
(73) Proprietor: Société des Produits Nestlé S.A., 1800 Vevey (CH)
(72) Inventor: SILVA ZOLEZZI, Irma, CH-1084 Carrouge (CH); MACE, Catherine, CH-1005 Lausanne (CH); GODFREY, Keith Malcolm, Ashurst Hampshire SO40 7AP (GB); BAKER, Philip Newton, Auckland 1011 (NZ); CHONG, Yap Seng, Singapore 268431 (SG)
(74) Representative: Krishnan, Sri
(86) International application number: PCT/EP2015/068196
(87) International publication number: WO 2016/020495

(56) References cited:
- EP-A1- 2 011 506
- EP-A1- 2 452 573
- EP-A1- 2 452 575
- WO-A1-2013/076121
- US-A- 5 763 392
- US-A1- 2005 112 211
- US-A1- 2010 111 915
- RAAKEL LUOTO ET AL: "Impact of maternal probiotic-supplemented dietary counselling on pregnancy outcome and prenatal and postnatal growth: a double-blind, placebo-controlled study", BRITISH JOURNAL OF NUTRITION, vol. 103, no. 12, 4 February 2010 (2010-02-04), pages 1792-1799, XP55030021, ISSN: 0007-1145, DOI: 10.1017/S0007114509993898
- KIRSI LAITINEN ET AL: "Probiotics and dietary counselling contribute to glucose regulation during and after pregnancy: a randomised controlled trial", BRITISH JOURNAL OF NUTRITION, vol. 101, no. 11, 19 November 2008 (2008-11-19), page 1679, XP55164118, ISSN: 0007-1145, DOI: 10.1017/S0007114508111461
- MARINE L. CROZE ET AL: "Chronic treatment with myo-inositol reduces white adipose tissue accretion and improves insulin sensitivity in female mice", THE JOURNAL OF NUTRITIONAL BIOCHEMISTRY, vol. 24, no. 2, 1 February 2013 (2013-02-01), pages 457-466, XP55212044, ISSN: 0955-2863, DOI: 10.1016/j.jnutbio.2012.01.008
- D'ANNA R ET AL: "Myo-inositol may prevent gestational diabetes in PCOS women", GYNECOLOGICAL ENDOCRINOLOGY, PARTHENON PUBLISHING, LONDON, GB, vol. 28, no. 6, 1 June 2012 (2012-06-01), pages 440-442, XP009181243, ISSN: 0951-3590, DOI: 10.3109/09513590.2011.633665

## Description

### Field of the invention

The present invention relates to the use of a combination of myo-inositol and one or more probiotic to minimise excessive fat accretion in a pregnant subject or a subject desiring to become pregnant, and/or to treat or prevent gestational diabetes mellitus and conditions associated therewith in a pregnant subject and/or its offspring.

### Background of the invention

Scientific evidence has accumulated showing that prenatal and post natal early nutrition and other environmental factors cause programming of long-term health and well-being, and can impact the risk of developing chronic diseases. Several studies have shown that changes in dietary intake or manipulation of individual macro and micronutrients during the reproductive period can have an impact in several physiological processes, such as growth, metabolism, appetite, cardiovascular function among others (Koletzko B et al (2011) Am J Nutr 94(s):2036-43S). Therefore nutritional status (nutrient stores and dietary intake) of women before and during pregnancy is of relevance to optimize neonatal and child health outcomes. Maternal nutrition is thought to affect the availability and supply of nutrients to the developing fetus that are required for critical developmental processes. The nutritional status of the woman before she gets pregnant also has an impact on her health and wellbeing, as well as on the health, growth and well-being of her baby. For example, micronutrient deficiencies have profound and often persistent effects on fetal tissues and organs, even in the absence of clinical signs of their deficiency in the mother (Ashworth CJ et al (2001) 122:527-35). Inadequate intakes of multiple micronutrients are common among women of reproductive age living in resource poor-settings (Torhem LE et al. (2010) J. Nutr. 140: 2051S-58S. Nutritional status of the lactating woman after delivery also has an impact on her health and wellbeing, as well as on the health, growth and well-being of her baby.

A large variety of nutrients have already been used in compositions for maternal administration and various nutritional compositions have been developed to address maternal nutrition needs. These typically contain vitamin and mineral mixes. However it would still be useful to specifically target the nutritional deficiencies of this specific population by selecting the most useful nutrients for these women such as to provide compositions tailored to the nutritional needs of women in the reproductive period to optimize the prenatal and postnatal nutrition of mothers, for the benefit of the mother and of her infant.

The use of probiotic bacteria for normalising plasma glucose concentration, increase insulin sensitivity and reduce the risk of development of gestational diabetes, in pregnant women, was disclosed in EP 2 011 506. Similarly, R Luoto et al: "Impact of maternal probiotic supplemented dietary counselling on pregnancy outcome on prenatal and postnatal growth: A randomized controlled trial" British Journal of Nutrition, Vol. 103, no. 12, 4 Feb. 2010 pages 1792-1799, and K Laitinen et al: "Probiotics and dietary counselling contribute to glucose regulation during and after pregnancy: a randomized controlled trial", British Journal of Nutrition, Vol. 101, No. 11, 19 Nov. 2008, page 1679, disclose that *lactobacillus rhamnosus* GG (CGMCC1.3724) and *bifidobacterium lactis* BB-12 (CNCM i-3446) may prevent gestational diabetes. US 5 763 392 discloses that a composition comprising myo-inositol may treat diabetes.

The present inventors have now discovered that a composition for maternal administration comprising myo-inositol together with probiotics is of particular usefulness to address the specific nutritional needs of women desiring to get pregnant, of pregnant and lactating women and of their offspring.

The present inventors have now discovered that a composition for maternal administration comprising myo-inositol together with probiotics is of particular usefulness to address the specific nutritional needs of women desiring to get pregnant, of pregnant and lactating women and of their offspring.

The inventors have also surprisingly found that the administration of a combination of myo-inositol and one or more probiotic to a subject may minimise excessive fat accretion (fat storage), and increase lean muscle mass in said subject. The effectiveness of this combination of ingredients is surprisingly better than could not be expected on the basis of the effects of these ingredients when used alone. This better effect may be more than additive.

As taught in in Athukorala C et al. BMC Pregnancy Childbirth, 2010, 10: 56; Ovesen P et al. Obstet Gynecol, 2011, 118: 305 and Aisling MM rat al. 2009, Diabetes Care 32(7): 1308), there is a well-established link between body fat deposition, obesity and GDM in pregnancy.

Accordingly, minimising fat accretion in a subject before and/or during pregnancy may prevent and/or treat GDM and/or prevent a condition associated with GDM in a pregnant subject or its offspring.

GDM is defined as any degree of glucose intolerance with onset or first recognition during pregnancy (Metzger BE, Coustan DR (Eds.): Proceedings of the Fourth International Work-shop-Conference on Gestational Diabetes Mellitus. Diabetes Care 21(Suppl. 2):B1- B167, 1998).

During pregnancy hormonal changes occur in a mother's body leading to increased insulin resistance and a higher glucose plasma concentration. These changes help ensure the transfer of nutrients from the mother to the fetus, thereby helping to ensure its optimal growth and development. Ordinarily the increased insulin resistance and higher glucose plasma concentrations are counteracted by increased insulin production by the mother. However, some mothers are not able to produce adequate amounts of insulin to counteract the changes and this can result in Gestational Diabetes Mellitus (hereinafter GDM) in said mothers.

GDM is a pregnancy disorder that can increase the risk of a number of maternal-fetal conditions, including macrosomia, birth injury, shoulder dystocia, premature delivery, and caesarian delivery (hereinafter C-section). Mothers suffering from GDM also have an increased risk of developing type II diabetes immediately after pregnancy and later in life. Also, the offspring of mothers suffering from GDM have an increased risk of developing an impaired glucose tolerance and/or suffering from excess weight/adiposity and associated metabolic disorders e.g. type II diabetes and obesity.

Accordingly, there is a need to find ways to treat and/or prevent GDM in pregnant subjects.

Further to the above, it has also been found that the administration of a combination of myo-inositol and one or more probiotic to a subject may increase the amount of insulin in the pancreas of said subject. This may result in a an improved insulin response to a glucose challenge and in consequence may treat or prevent GDM or a condition associated therein in a pregnant subject or it's offspring.

### Summary of the invention:

The invention is set out in the claims. As stated above, the present inventors have now discovered that a composition for maternal administration comprising myo-inositol together with probiotics is of particular usefulness to address the specific nutritional needs of women desiring to get pregnant, of pregnant and lactating women and of their offspring. An object of the present invention is the use of said composition to minimise excessive fat accretion in a subject before and/or during pregnancy and/or to treat or prevent GDM in a pregnant subject e.g. a pregnant woman, cat or dog.

As stated hereinabove, GDM is associated with a variety of conditions affecting the pregnant subject and/or its offspring e.g. preterm and caesarian delivery, birth injury to the mother or baby, shoulder dystocia, macrosomia, excessive offspring blood glucose concentration, excess weigh/adiposity and associated metabolic disorders e.g. type II diabetes, fatty liver disease and obesity, immediately after birth and later in the life of the offspring, and an increased risk for the mother of having or developing type 2 diabetes immediately after birth and later in life. Accordingly, by treating or preventing GDM the combination of myo-inositol and one or more probiotic may also be used to treat or prevent these conditions in these pregnant subjects or their offspring.

The combination of myo-inositol and one or more probiotic can be particularly effective, at minimizing fat accretion in a subject before and/or during pregnancy and/or at preventing and/or treating GDM, when further combined with vitamin B2 and/or one or more of vitamins B6, B12 and D.

The combination of myo-inositol and one or more probiotic, optionally further combined with one or more of vitamins B2, B6, B12 and D, may be administered enterally to a subject before and/or during pregnancy and/or during lactation.

The combination of myo-inositol and one or more probiotic optionally further combined with one or more of vitamin B2, B6, B12 and D, may be administered or employed in any form suitable for ingestion by a subject e.g. in the form of a powdered nutritional composition to be reconstituted in for example milk, juice or water, a food product, a drink, a nutritional supplement such as a powdered nutritional supplement to be sprinkled on food or dissolved in an aqueous medium for example milk, juice or water, or a nutraceutical.

Myo-inositol and one or more probiotic, optionally further combined with one or more of vitamin B2, B6, B12 and D, may be used in the manufacture of a medicament for use to minimise excessive fat accretion in a subject before and/or during pregnancy and/or to treat or prevent GDM in a pregnant subject.

Myo-inositol and one or more probiotic and optionally one or more of vitamin B2, B6, B12 and D, may be provided along with a label indicating dosage requirements in a kit for use to minimise excessive fat accretion in a subject before and/or during pregnancy and/or to treat or prevent GDM in a pregnant subject.

### Drawings

Figure 1 is a graph showing the percentage of the surveyed women from example 3 found to have deficiencies in vitamin D, vitamin B12, vitamin B6, folate, iron and Zinc respectively.
Figure 2 is a chart illustrating the effect of a combination of myo-inositol and probiotics on the delta % fat mass of rats after 10 weeks of treatment in non - pregnant Goto Kakizaki rats.
Figure 3 is a chart illustrating the effect of a combination of myo-inositol and probiotics on the ratio of fat gain to weight gain in non - pregnant Goto Kakizaki rats after 10 weeks of treatment.
Figure 4 is a chart illustrating the effect of a combination of myo-inositol and probiotics on the amount of insulin in the pancreas of at day 19.5 of gestation in pregnant Goto Kakizaki rats.

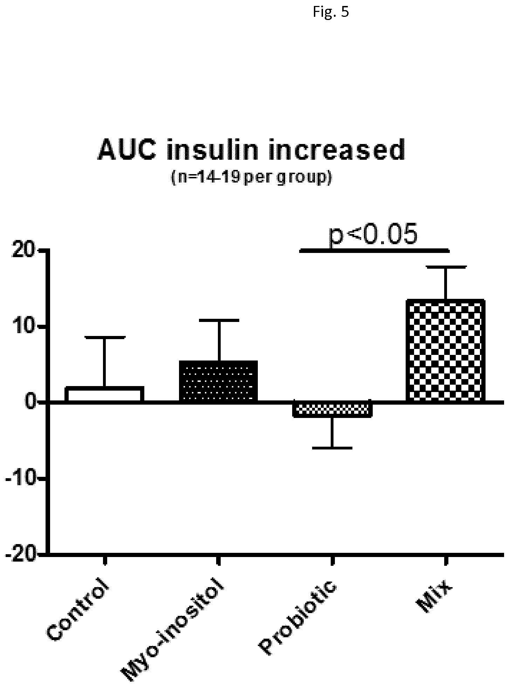

Figure 5 is a chart illustrating the effect of a combination of myo-inositol and probiotics on the AUC insulin increase at day 16.5 of gestation in pregnant Goto Kakizaki rats.
Figure 6 is a chart illustrating the effect of a combination of myo-inositol and probiotics on SLC5A11 transcription in gastrocnemius.

### Detailed Description

The present inventors have investigated the specific needs of women desiring to get pregnant, and of pregnant and of lactating women to identify the most useful nutrients for this specific population. Based on the finding above and these studies, they have designed an optimized composition comprising myo-inositol and probiotics to specifically address the most acute needs of this specific population.

Inositol or cyclohexane-1,2,3,4,5,6-hexol is a polyol existing under nine stereoisomeric forms depending on the spatial orientation of its six hydroxyl groups. Myo-Inositol, or cis-1,2,3,5-trans-4,6-cyclohexanehexol, is the predominant isomeric form of inositol. Myo-Inositol is a compound present in animal and plant cells and plays an important role in various cellular processes, as the structural basis for secondary messengers in eukaryotic cells, in particular as inositol triphosphates (IP3), phosphatidylinositol phosphate lipids (PIP2/PIP3) and inositol glycans. Myo-inositol has been shown to participate in a variety of biological process such as cell growth and survival, development and function of peripheral nerves, osteogenesis, energy metabolism and reproduction (Croze et al. (2013) Biochimie 95:1811-1827). Myo-inositol is found as free-from, phosphoinositides and phytic acid, in fresh fruits and vegetables, and in all foods containing seeds (beans, grains and nuts) (Clements RS and Darnell B. Am J Clin Nutr (1980) 33:1954-1967). Myo-Inositol from phytic acid can be released in the gut by the enzymes phytases found in plants, microorganisms and in animal tissues (Schlemmer U et al. Mol Nutr Food Res (2009) 53:S330-S375). These enzymes are capable of releasing free inositol, orthophosphate, and intermediary products including the mono-, di-, tri-, tetra- and penta-phosphate forms of inositol. Much of the ingested inositol hexaphosphate is hydrolysed to inositol.

Due to its important physiological role, and in particular due to its ability to promote cellular growth, myo-inositol is of particular interest for women desiring to get pregnant and for pregnant and lactating women. However, the dietary intake study carried out by the inventors and detailed in Example 4 has shown that pregnant women usually do not take enough of the kind of food in which myo-inositol is found, mostly fruits and vegetables. The present inventors have therefore found that it is of particular benefit for women desiring to get pregnant, for pregnant women and for lactating women to get supplementation in myo-inositol.

The myo-inositol is preferably provided in an amount of 0.2 to 5 g, preferably 1.5 to 5 g, more preferably 2 to 5 g, most preferably 2 to 4 g per daily dose.

The term myo-inositol as used herein refers to myo-inositol (cis-1,2,3,5-trans-4,6-cyclohexanehexol) and /or a metabolite thereof.

The term metabolite as used herein refers to any substance produced and/or formed by the body of a subject from a particular compound after its administration e.g. ingestion. It includes active forms and catabolites of a compound.

Any source of myo-inositol suitable for ingestion by the pregnant subject may be used in the invention.

A metabolite of myo-inositol can be selected from the group consisting of D-chiro-inositol, L-chiro-inositol and a combination of the foregoing. In particular the metabolite is D-chiro-inositol.

It is also particularly beneficial to combine myo-inositol with probiotics, as these have been found to improve the gut barrier function and to help nutrients pass through the gut (Cani PD et al. (2009) Gut 58:1091-1103). Combining myo-inositol with probiotics thus enhances the absorption of myo-inositol and other nutrients that may be present in the composition.

The term probiotic as used herein refers to live probiotic bacteria, non-replicating probiotic bacteria, dead probiotic bacteria, non-viable probiotic bacteria, fragments of probiotic bacteria such as DNA, metabolites of probiotic bacteria, cytoplasmic compounds of probiotic bacteria, cell wall materials of probiotic bacteria, culture supernatants of probiotic bacteria, and combinations of any of the foregoing.

In particular the probiotic is live probiotic bacteria non-replicating probiotic bacteria, dead probiotic bacteria, non-viable probiotic bacteria, and any combination thereof. More particularly the probiotic is live probiotic bacteria.

The probiotics comprise a combination of Lactobacillus and Bifidobacterium. The most preferred Lactobacillus strain is the Lactobacillus rhamnosus GG strain deposited by Nestle R&D centre Shanghai Ltd (13 Qiao Nan, Cao An Road, Jiading District, Shanghai 201812, P.R. China) at the China General Microbiological Culture Collection Centre (CGMCC) and available under the deposit number CGMCC 1.3724. The most preferred Bifidobacterium strain is the Bifidobacterium lactis BB12 strain deposited at the Collection Nationale de Cultures De Microorganismes (CNCM)as CNCM 1-3446. Preferably the probiotics comprise a mixture of the Lactobacillus rhamnosus GG strain available under the deposit number CGMCC 1.3724 and of the Bifidobacterium lactis BB12 strain deposited as CNCM 1-3446. Most preferably the probiotics consist of a mixture of the Lactobacillus rhamnosus GG strain available under the deposit number CGMCC 1.3724 and of the Bifidobacterium lactis BB12 strain deposited as CNCM 1-3446. In a preferred embodiment, the probiotic is provided in an amount of from 105 to 1012 colony forming units (cfu) per daily dose, more preferably from 107 to 1011 cfu per daily dose.

In a preferred embodiment, the composition of the invention further comprises at least one vitamin selected from vitamin B2, vitamin B6, vitamin B12, vitamin D and mixtures thereof. Preferably the composition comprises vitamin B2, vitamin B6, vitamin B12 and vitamin D.

In the study detailed in Example 3, the present inventors have surprisingly shown that pregnant women were more often deficient in vitamins B6, B12 and D compared to other nutrients. Also, in the study detailed in Example 4, they have demonstrated that vitamin B2 was not consumed in sufficient amounts by a significant proportion of the pregnant woman population. It is therefore of particular interest to supplement the diet of pregnant women with these vitamins in order to compensate these particularly often-occurring deficiencies.

The term vitamin B2 as used herein refers to vitamin B2 and /or a metabolite thereof.

Any source of Vitamin B2 suitable for ingestion in the pregnant subject may be used. In particular, vitamin B2 may be riboflavin e.g. riboflavin sold under the trademark Riboflavin Universal.

A metabolite of vitamin B2 can be selected from the group consisting of flavin mononucleotide (hereinafter FMN), Flavin Adenine Dinucleotide (hereinafter FAD), and salts thereof e.g. riboflavin-5'-phosphate sodium salt.

The term vitamin B12 as used herein refers to vitamin B12 and /or a metabolite thereof.

The term vitamin B6 as used herein refers to vitamin B6 and /or a metabolite thereof.

The term vitamin D as used herein refers to vitamin D, a precursor thereof, and /or a metabolite thereof.

The term precursor as used herein refers to any substance administered e.g. ingested by a subject and used by the body of said subject to produced and/or form a particular compound.

In particular the vitamin B6 may be pyroxidine hydrochloride, and/or a metabolite of vitamin B6 selected from the group consisting of pyridoxal 5'-phosphate (hereinafter PLP).

In particular the vitamin B12 may be cyanocobalamin, and/or a metabolite of vitamin B12 selected from the group consisting of hydroxocobalamin, methylcobalamin, adenosylcobalamin, and a combination thereof.

In particular, the vitamin D may be vitamin D2, vitamin D3 or a combination thereof, a precursor of vitamin D selected from the group consisting of 7-dehydrocholecalciferol, a metabolite of vitamin D selected from the group consisting of 25-hydroxyvitamin D3, 1,25-dihydroxyvitamin D3, 25-hydroxyvitamin D2, 1,25-dihydroxyvitamin D2, and a combination of the foregoing.

Most preferably the vitamin D is vitamin D3, a metabolite of vitamin D selected from the group consisting of 25-hydroxyvitamin D3, 1,25-dihydroxyvitamin, and combination of any of the foregoing. Even more particularly the vitamin D3 is cholecalciferol.

If present, the vitamin B2 is preferably provided in an amount of from 0.14 to 14 mg per daily dose. If present the vitamin B6 is preferably provided in an amount of from 0.19 to 19 mg per daily dose. If present, the vitamin B12 is preferably provided in an amount of from 0.26 to 26 µg per daily dose. If present, the vitamin D is preferably provided in an amount of from 1.5 to 100 µg per daily dose. Most preferably the composition of the invention comprises 1.8 mg of vitamin B2, 2.6 mg of vitamin B6, 5.2 µg of vitamin B12 and 10 µg of vitamin D per daily dose.

The study of Example 3 has also provided evidence of common zinc deficiencies in at least certain populations of pregnant woman, even though in a lesser extent than vitamins B2, B12, B6 and D. It is therefore also particularly advantageous to supplement the diet of pregnant women with zinc. Thus, in a preferred embodiment the composition of the invention further comprises zinc. More preferably zinc is present in an amount of from 1.1 to 40 mg per daily dose.

Any source or form of zinc suitable for ingestion by the pregnant subject may be used. In particular the zinc may be a zinc salt such as zinc chloride, zinc picolinate, zinc sulfate, zinc oxide, zinc acetate, zinc carbonate, and combinations of the foregoing.

More particularly the zinc is zinc chloride.

Additional vitamins and minerals may also be added. For example, vitamins and minerals may be added in accordance with the recommendations of Government bodies such as the USRDA. For example, the composition may contain one or more of the following micronutrients, calcium, magnesium, phosphorus, iron, zinc, copper, iodine, selenium, vitamin A or retinol activity equivalent (RAE) for example in the form of beta carotene or a mix of carotenoids, Vitamin C, Vitamin B1, niacin, folic acid, biotin, Vitamin E. Preferably the composition may contain one or more of the following micronutrients in the following amounts: 100 to 2500 mg calcium, 35 to 350 mg magnesium, 70 to 3500 mg phosphorus, 2.7 to 45 mg iron, 1.1 to 40 mg zinc, 0.1 to 10 mg copper, 22 to 1,100 µg iodine, 6 to 400 µg selenium, 77 to 3000 µg of vitamin A or retinol activity equivalent (RAE) for example in the form of beta carotene or a mix of carotenoids, 8.5 to 850 mg Vitamin C, 0.14 to 14 mg Vitamin B1, 1.8 to 35 mg niacin, 60 to 1000 µg folic acid, 3 to 300 µg biotin, 1.9 to 109 µg Vitamin E.

The composition of the invention may advantageously further comprise at least one oil selected from long chain polyunsaturated fatty acids, such as arachidonic acid (ARA), eicosapentaenoic acid (EPA) and/or docosahexaenoic acid (DHA), in any suitable amount as known by the person skilled in the art for example in an amount of 100-500 mg per daily dose, more preferably between 200 and 400 mg per daily dose.

However, a high fat intake and/or Maternal PUFA supplementation during pregnancy has been linked to an increased risk of higher adiposity in the offspring. Accordingly, in a particular embodiment the composition of the invention does not comprise a long chain polyunsaturated fatty acid and/or animal fats and/or vegetable fats.

In another particular embodiment the composition of the invention does not comprise an oligosaccharide.

Any of the nutrients mentioned herein may be used in any amount that is effective in achieving the objective of the present invention. Skilled artisans will be able to determine appropriate dosages. Typically, dosage will depend on age, size and health status of the mother, on her lifestyle, as well as on her genetic heritage.

Typically, amounts are defined in the present application as amounts per daily dose. The amount of nutrient may thus be selected in each composition accordingly depending upon whether it is intended to be consumed once a day or more or less frequently.

The nutrients may be provided as a sustained release formulation. This way, the nutrient can be consumed less frequently, while the body is still constantly supplied with sufficient amount of such nutrient.

The composition of the present invention is intended for maternal administration. This means that the composition is to be administered to a woman desiring to get pregnant, to a pregnant woman and/or to a lactating woman. Preferably, it is to be administered to a woman desiring to get pregnant and/or to a pregnant woman, most preferably to a pregnant woman.

Preferably, the composition of the invention is administered to a woman desiring to get pregnant, for example during at least 1, 2, 3 or 4 months preceding the pregnancy or desired pregnancy. When the composition is to be administered to a pregnant or lactating woman, the composition is preferably administered for at least 4, preferably at least 8, more preferably at least 12, more preferably at least 16, more preferably at least 20, more preferably at least 24, more preferably at least 28, even more preferably at least 36 weeks during pregnancy and or lactation. As the nutritional requirements increase in the second and third trimester of pregnancy, it is further preferred to administer the composition of the invention throughout the third trimester of pregnancy and most preferably throughout the second and third trimesters of pregnancy.

It is important that women desiring to get pregnant prepare their body for pregnancy by taking appropriate nutrients. Then, women's nutrient needs increase during pregnancy and lactation. Also lactation is demanding on maternal stores of energy, protein and other nutrients that need to be established and replenished. The composition of the present invention is specifically designed to meet the needs of the woman during each of these periods.

The composition can be in any form that is suitable to administer all the ingredients to the woman. For example it can be in the form of a powdered nutritional composition to be reconstituted in milk or water, a food product, a functional food product, a drink, (beverage), a dairy product, a pharmaceutical formulation, a pet food product, a nutritional supplement or a nutraceutical.

When the composition is in the form of a powdered nutritional composition to be reconstituted in milk or water it preferably comprises a protein source, a carbohydrate source and a lipid source, preferably together with lecithin. More preferably it also comprises soya lecithin and/or a bulking agent. The protein source may be dried milk or dried skimmed milk. As carbohydrate source sucrose and/or maltodextrin may be used. The lipid source may be vegetable oil. The formulation may also alternatively or additionally contain glucose syrup, milk fat, magnesium citrate, choline salts and esters, prebiotic fibers, and/or ascorbyl palmitate. Flavor compounds, such as cocoa powder or honey, for example, may be added to provide taste variations.

The term "food product", as used herein, refers to any kind of product that may be safely consumed by a human or animal. Said food product may be in solid, semi-solid or liquid form and may comprise one or more nutrients, foods or nutritional supplements. For instance, the food product may additional comprise the following nutrients and micronutrients: a source of proteins, a source of lipids, a source of carbohydrates, vitamins and minerals. The composition may also contain anti-oxidants, stabilizers (when provided in solid form) or emulsifiers (when provided in liquid form).

The term "functional food product" as used herein, refers to a food product providing an additional health-promoting or disease-preventing function to the individual.

Food products and functional food products include for example cereal-based products, yogurts or other milk-derived products and bars.

The term "nutritional supplement", or "dietary supplement", as used herein, refers to a nutritional product that provides nutrients to an individual that may otherwise not be consumed in sufficient quantities by said individual. For instance, a nutritional supplement may include vitamins, minerals, fiber, fatty acids, or amino acids.

Supplements can for example be provided in the form of a pill, a tablet, a lozenger, a chewy capsule or tablet, a tablet or capsule, or a powder supplement that can for example be dissolved in water or sprinkled on food. Most preferred is a powder supplement that can be dissolved in liquid or sprinkled on food, most preferably dissolved in water. Such supplements typically provide the selected nutrients while not representing a significant portion of the overall nutritional needs of the subject. Typically they do not represent more than 0.1%, 1%, 5%, 10% or 20% of the daily energy need of the subject.

The term "dairy products", as used herein, refers to food products produced from animals such as cows, goats, sheep, yaks, horses, camels, and other mammals. Examples of dairy products are low-fat milk (e.g. 0.1%, 0.5% or 1.5% fat), fat-free milk, milk powder, whole milk, whole milk products, butter, buttermilk, buttermilk products, skim milk, skim milk products, high milk-fat products, condensed milk, crème fraiche, cheese, ice cream and confectionery products, probiotic drinks or probiotic yoghurt type drinks.

The term "pharmaceutical formulation" as used herein, refers to a composition comprising at least one pharmaceutically active agent, chemical substance or drug. The pharmaceutical formulation may be in solid or liquid form and can comprise at least one additional active agent, carrier, vehicle, excipient, or auxiliary agent identifiable by a person skilled in the art. The pharmaceutical formulation can be in the form of a tablet, capsule, granules, powder, liquid or syrup. The term "beverage product" as used herein, refers to a nutritional product in liquid or semi-liquid form that may be safely consumed by an individual.

The term "pet food product" as used herein refers to a nutritional product that is intended for consumption by pets. A pet, or companion animal, as referenced herein, is to be understood as an animal selected from dogs, cats, birds, fish, rodents such as mice, rats, and guinea pigs, rabbits, etc.

All ingredients of the composition can be admixed together.

As stated above, in addition to the present inventors identifying that a composition for maternal administration comprising myo-inositol together with probiotics is of particular usefulness to address the specific nutritional needs of women desiring to get pregnant, of pregnant and lactating women and of their offspring, said inventors have also surprisingly found that the administration of a combination of myo-inositol and one or more probiotic to a subject may minimise excessive fat accretion in a subject before and/or during pregnancy and/or treat or prevent GDM in a pregnant subject.

Accordingly, in another aspect of the present invention there is provided a combination of myo-inositol and one or more probiotic for use to prevent or minimise excessive fat accretion in a subject before and/or during pregnancy and/or to treat or prevent GDM and a condition associated therewith in a pregnant subject and/or its offspring.

The term "subject" as used herein refers to a mammal and more particularly a cat, a dog or a human.

A mammal is considered overweight or obese if there body fat percentage is too high.

A human female is considered obese if there body fat % is greater than 37%.

A human female is considered overweight if there body fat % is 31 to 36%.

Excess fat accretion during pregnancy has been linked to an increased risk of developing gestational complications e.g. GDM and undesirable outcomes in offspring. Accordingly, minimsing excessive fat accretion pre- pregnancy and during pregnancy may be beneficial, further, as stated above, excess fat accretion is linked to GDM.

What constitutes excess fat accretion during pregnancy will depend on the weight and body composition e.g. % fat, of the subject. The skilled person will be able to determine this based on clinical health care guidelines.

Body fat % can be measured by carrying out a bioelectrical impedance analysis (hereinafter BIA) on the subject, or by subjecting the subject to nuclear magnetic resonance (hereinafter NMR).

The term "GDM" as used herein refers to any degree of impaired glucose tolerance that onsets or is first recognized during pregnancy.

Whether or not a mammal has an impaired glucose tolerance may be determined by measuring its fasting glucose plasma concentration, or by carrying out an oral glucose tolerance test (OGTT). The skilled person will be familiar with these tests and the criteria for diagnosing an impaired glucose tolerance and hence GDM. According to the criteria set out in the National Academy of clinical biochemistry (NACB) guidelines and the international association of diabetes and pregnancy study group (IADPSG) guidelines, published by the American Association for clinical chemistry (AACC), a pregnant human subject is considered as having an impaired glucose tolerance if their fasting plasma glucose concentration equates to 5.1mmol/L or more, or if their blood glucose concentration is higher than 10 mmol/L 1hour after a 75gram glucose drink, or higher than 8.5 mmol/L 2 hours after a 75gram glucose drink.

The term "treat" as used herein also encompasses amelioration and/or alleviation of a condition i.e. the amelioration and/or alleviation of the symptoms of a condition. It may for example encompass the reduction of the severity of a condition in a subject.

The term "prevent" as used herein refers to the prevention of the occurrence, or reduction of the risk of the occurrence, of a condition in a subject.

Any source of myo-inositol suitable for ingestion by the pregnant subject may be used in the invention.

A metabolite of myo-inositol can be selected from the group consisting of D-chiro-inositol, L-chiro-inositol and a combination of the foregoing. In particular the metabolite is D-chiro-inositol.

In particular the probiotic is live probiotic bacteria non-replicating probiotic bacteria, dead probiotic bacteria, non-viable probiotic bacteria, and any combination thereof. More particularly the probiotic is live probiotic bacteria.

The one or more probiotic may be any lactic acid bacteria, Bifidobacteria, or combination thereof, wherein said lactic acid bacteria and Bifidobacteria have established probiotic characteristics. Non limiting examples of lactic acid bacteria strains include; Lactobacillus rhamnosus ATCC 53103 obtainable inter alia from Valio Oy of Finland under the trade mark LGG and Lactobacillus rhamnosus deposited by Nestle R&D centre Shanghai Ltd (13 Qiao Nan, Cao An Road, Jiading District, Shanghai 201812, P.R. China) at the China General Microbiological Culture Collection Centre (CGMCC) and available under the deposit number CGMCC 1.3724. None limiting examples of Bifidobacteria strains include; Bifidobacterium lactis deposited at the Collection Nationale de Cultures De Microorganismes (CNCM) CNCM 1-3446 sold inter alia by the Christian Hansen company of Denmark under the trade mark Bb12, Bifidobacterium longum ATCC BAA-999 sold by Morinaga Milk Industry Co. Ltd. of Japan under the trade mark BB536, the strain of Bifidobacterium breve sold by Danisco under the trade mark Bb-03, the strain of Bifidobacterium breve sold by Morinaga under the trade mark M-16V and the strain of Bifidobacterium breve sold by Institut Rosell (Lallemand) under the trade mark R0070.

In a particular embodiment the one or more probiotic is a mixture of Lactobacillus rhamnosus CGMCC 1.3724 and Bifidobacterium lactis CNCM I-3446

The Lactobacillus rhamnosus CGMCC 1.3724 and Bifidobacterium lactis CNCM I-3446 can be used in a ratio of 1:99 to 99:1, however, more particularly they will be used in a ratio of 1:2 to 2 to 1, even more particularly 1:1.

The combination of myo-inositol and one or more probiotic may be particularly effective at minimizing fat accretion in a subject before and/or during pregnancy and/or at treating or preventing GDM and preventing a condition associated with GDM in a pregnant subject or its offspring, when further combined with one or more of vitamin B2, B6, B12 and D. The combination of myo-inositol and one or more probiotic when further combined with one or more of these listed ingredients may provide an improved effect over what could be expected on the basis of the effect of the myoinositol and one or more probiotics, or one or more of vitamins B2, B6, B12 and D when used alone to minimise excessive fat accretion in a subject before and/or during pregnancy and/or to treat or prevent GDM in a pregnant subject. An improved effect may be a more than additive or synergistic effect.

Accordingly, in an embodiment the combination of myo-inositol and one or more probiotic is further combined with one or more of vitamin B2, B6, B12 and D.

In particular the myo-inositol and one or more probiotic is further combined with vitamin B2, B6, B12 and D.

Any source of Vitamin B2 suitable for ingestion in the pregnant subject may be used. In particular, vitamin B2 may be riboflavin e.g. riboflavin sold under the trademark Riboflavin Universal.

A metabolite of vitamin B2 can be selected from the group consisting of flavin mononucleotide (hereinafter FMN), Flavin Adenine Dinucleotide (hereinafter FAD), and salts thereof e.g. riboflavin-5'-phosphate sodium salt.

Any source of vitamin B6, vitamin B12 and vitamin D suitable for ingestion in the pregnant subject may be used in the invention.

In particular the vitamin B6 may be pyroxidine hydrochloride, and/or a metabolite of vitamin B6 selected from the group consisting of Pyridoxal 5'-phosphate (hereinafter PLP).

In particular the vitamin B12 may be cyanocobalamin, and/or a metabolite of vitamin B12 selected from the group consisting of hydroxocobalamin, methylcobalamin, adenosylcobalamin, and a combination thereof.

In particular, the vitamin D may be vitamin D2, vitamin D3 or a combination thereof, a precursor of vitamin D selected from the group consisting of 7-dehydrocholecalciferol, a metabolite of vitamin D selected from the group consisting of 25-hydroxyvitamin D3, 1,25-dihydroxyvitamin D3, 25-hydroxyvitamin D2, 1,25-dihydroxyvitamin D2, and a combination of the foregoing.

More particularly the vitamin D is vitamin D3, a metabolite of vitamin D selected from the group consisting of 25-hydroxyvitamin D3, 1,25-dihydroxyvitamin, and combination of the foregoing. Even more particularly the vitamin D3 is Cholecalciferol.

The combination of myo-inositol and one or more probiotic optionally further combined with one or more of vitamin B2 B6, B12 and D, can be employed in any effective dose that provides a benefit with respect to minimising fat accretion in a subject before and/or during pregnancy and/or with respect to the treatment or prevention of GDM and a condition associated therewith in a pregnant subject and/or its offspring.

With respect to fat accretion, an effective dose may be any dose that lowers fat accretion in comparison to when said dose is not administered.

With respect to GDM, an effective dose may be any dose that improves, by any degree, an impaired glucose tolerance in a pregnant subject.

It is well within the purview of the skilled person to determine an effective dose. Typically, an effective dose will depend on age, size and health status of the subject, on the subject's lifestyle, as well as on its genetic heritage.

With respect to minimising fat accretion, an effective dose may, for example, be determined by carrying out a bioelectrical impedance analysis (hereinafter BIA) on the subject, or by subjecting the subject to nuclear magnetic resonance (hereinafter NMR), before and after taking said dose for a certain period of time. The period may be 10 days to 3 months.

With respect to GDM, an effective dose may, for example, be determined by testing the effect of a dose on a subject's fasting glucose plasma concentration, or by carrying out an OGTT. An effective dose should improve a subject's fasting glucose concentration and lower a subject's glycemic response.

A particularly useful dose of myo-inositol is from 0.2 to 5mg, 1.5 to 5mg, 2 to 4g, or 2g.

A particularly useful dose of the one or more probiotic bacteria is from 10e5 to 10e12 colony forming units (cfu), from 10e7 to 10e11 cfu, 10e9cfu.

A particularly useful dose of vitamin B2, if present, may be 0.14 to 14 mg, 1 to 2.5mg, 1.5 to 2mg, or 1.8mg.

A particularly useful dose of vitamin B6, if present, is 0.19 to 19 mg, or 2.6mg. A particularly useful dose of vitamin B12, if present, is 0.26 to 26µg, or 5.2µg. A particularly useful dose of vitamin D, if present, is 1.5 to 100 µg, 5 to 50 µg or 10µg.

The term "dose" as used herein refers to a daily quantity that is administered to a subject before and/or during pregnancy and/or during lactation. The daily quantity or dose may be administered all at once or it may be spread out over several administrations throughout a day. The dose may be by administered by any known method, in particularly enterally e.g. orally.

The dose(s) may be administered at any time e.g. day time or night time. The dose may be particularly effective if administered before the subject has food or beverage e.g. has a meal or a snack.

In an embodiment the dose is spread over 2 administrations, in a particular the dose is spread over 2 administrations, one in the morning and one in the evening, in particularly before breakfast and before the evening meal.

The term "breakfast" as used herein refers to the first meal of the day.

The term "evening meal" as used herein refers the last meal of the day.

In another aspect of the present invention there is provided a kit for for use to minimise excessive fat accretion in a subject before and/or during pregnancy and/or to treat or prevent GDM and a condition associated therewith in a pregnant subject and/or its offspring, the kit comprising:
- Myo-inositol and one or more probiotic and optionally one or more of vitamin B2, B6, B12 and D
- A label indicating dosage requirements for a pregnant subject.

The dosage requirements may be 2 administrations per day, in particular 2 administrations per day before any food or beverage. More particularly, 2 administrations per day wherein one is in the morning and one is in the evening. Even more particularly, 2 administrations per day wherein one is in the morning before breakfast and one is in the evening before the evening meal.

The myo-inositol, one or more probiotic, and the optional one or more of vitamin B2, B6, B12 and D may all be provided in a format providing sustained release of said vitamins or one or more probiotic. This way, these compounds can be consumed less frequently, while the body is still constantly supplied with a sufficient amount of them.

The combination of myo-inositol and one or more probiotic optionally further combined with one or more of vitamin B2, B6, B12 and D, may be administered in the same composition. Alternatively, the myo-inositol and one or more probiotic and optional one or more of vitamin B2, B6, B12 and D, may be administered separately to one another or a combination of each other e.g. myo-inositol (optionally with one or more of vitamin B2, B6, B12 and D) may be administered separately to said one or more probiotic. The separate administration may in particular be simultaneously but separate administration e.g. myo-inositol (optionally with one or more of vitamin B2, B6, B12 and D) may be administered at the same time but separately to said one or more probiotic. e.g. in two tablets administered separately and sequentially in quick succession e.g. within 5, 4, 3, 2, 1 minute, to said one or more probiotic.

The combination of myo-inositol and one or more probiotic optionally further combined with one or more of vitamin B2 B6, B12 and D, is intended for administration to a subject desiring to get pregnant, to a pregnant subject and/or to a lactating subject. In particular it is to be administered to a subject desiring to get pregnant and/or to a pregnant subject. More particularly it will be administered to a pregnant subject.

If the combination of myo-inositol and one or more probiotic optionally further combined with one or more of vitamin B2, B6, B12 and D, is administered to a subject desiring to get pregnant it may for example be administered during at least 1, 2, 3 or 4 months preceding the pregnancy or desired pregnancy. If the combination of myo-inositol and one or more probiotic optionally further combined with one or more of vitamin B2, B6, B12 and D, is administered to a pregnant subject, it may be administered throughout or partially throughout the pregnancy e.g. for at least 4, at least 8, at least 12, at least 16, at least 20, at least 24, at least 28, or at least 36 weeks depending on the gestational period of the subject. Administration may also continue throughout or partially throughout the lactation period of said subject.

Since the risk of GDM increases in the second and third trimester of pregnancy, administration may be particularly beneficial in the second and third trimester of pregnancy for the prevention or treatment of GDM, or the prevention of a condition associated therewith in a pregnant subject or its offspring.

The combination of myo-inositol and one or more probiotic optionally further combined with one or more of vitamin B2, B6, B12 and D, may be administered or employed in any form suitable for ingestion by the subject e.g. it can be employed in the form of a composition e.g. a powdered nutritional composition, a food product, a functional food product, a drink (beverage), a dairy product, a pharmaceutical formulation, a pet food product, a nutraceutical, a nutritional supplement e.g. a powdered nutritional supplement e.g. to be sprinkled on food or dissolved in an aqueous medium e.g. water or juice, or milk.

When the composition is in the form of a powdered nutritional composition it may further comprises a protein source, a carbohydrate source and a lipid source, possibly together with lecithin. It may also comprise soya lecithin and/or a bulking agent. The protein source may be dried milk or dried skimmed milk. As carbohydrate source sucrose and/or maltodextrin may be used. The lipid source may be vegetable oil. The formulation may also alternatively or additionally contain glucose syrup, milk fat, magnesium citrate, choline salts and esters, prebiotic fibers, and/or ascorbyl palmitate. Flavor compounds, such as cocoa powder or honey, for example, may be added to provide taste variations.

The combination of myo-inositol and one or more probiotic optionally further combined with one or more of vitamin B2, B6, B12 and D, may also be used in combination with other vitamins and minerals. For example, vitamins and minerals recommended by a governmental body, such as USRDA, for supplementation in pregnancy e.g. calcium, magnesium, phosphorus, iron, zinc, copper, iodine, selenium, vitamin A or retinol activity equivalent (RAE) e.g. beta carotene or a mix of carotenoids, vitamin C, vitamin B1, niacin, folic acid, biotin, vitamin E.

It may be particularly beneficial if the combination of myo-inositol and one or more probiotic optionally further combined with one or more of vitamin B2, B6, B12 and D, is used in combination with one or more of the following micronutrients in the following amounts: 100 to 2500 mg calcium, 35 to 350 mg magnesium, 70 to 3500 mg phosphorus, 2.7 to 45 mg iron, 1.1 to 40 mg zinc, 0.1 to 10 mg copper, 22 to 1,100 µg iodine, 6 to 400 µg selenium, 77 to 3000 µg of vitamin A or retinol activity equivalent (RAE) for example in the form of beta carotene or a mix of carotenoids, 8.5 to 850 mg vitamin C, 0.14 to 14 mg Vitamin B1, 1.8 to 35 mg niacin, 60 to 1000 µg folic acid, 3 to 300 µg biotin, 1.9 to 109 µg Vitamin E.

The combination of myo-inositol and one or more probiotic optionally further combined with one or more of vitamin B2, B6, B12 and D, may also be advantageously used in combination with at least one oligosaccharide and/or at least one long chain polyunsaturated fatty acids, such as arachidonic acid (ARA), eicosapentaenoic acid (EPA) and/or docosahexaenoic acid (DHA). The oligosaccharides and long chain polyunsaturated fatty acids can be used in any concentration safe for administration to the subject.

The combination of myo-inositol and one or more probiotic optionally further combined within one or more of vitamin B2, B6, B12 and D, may also be used in combination with other ingredients commonly used in the form of composition in which it is employed e.g. a powdered nutritional supplement, or a food product. Non limiting examples of such ingredients include: other nutrients, for instance, selected from the group of lipids (optionally in addition to DHA and ARA), carbohydrates, and protein, micronutrients (in addition to those set out above), or pharmaceutically active agents; conventional food additives such as anti-oxidants, stabilizers, emulsifiers, acidulants, thickeners, buffers or agents for pH adjustment, chelating agents, colorants, excipients, flavor agents, osmotic agents, pharmaceutically acceptable carriers, preservatives, sugars, sweeteners, texturizers, emulsifiers, water and any combination thereof. However, notwithstanding the above, a high fat intake during pregnancy and/or Maternal PUFA supplementation has been linked to an increased risk of higher adiposity in the offspring. Accordingly, in a particular embodiment myo-inositol and one or more probiotic optionally further combined with one or more of vitamin B2, B6, B12 and D is not used in combination with a long chain polyunsaturated fatty acid and/or animal fats and/or vegetable fats.

In a particular embodiment the myo-inositol and one or more probiotic optionally further combined with one or more of vitamin B2, B6, B12 and D is not used in combination with an oligosachharide.

As stated hereinabove, GDM is associated with a variety of conditions that can affect the pregnant subject and/or its offspring. GDM is considered as being associated with a condition if it increases the risk of the development of that condition during pregnancy, during birth, after birth or later in the life of said pregnant subject or its offspring.

Non limiting examples of conditions associated with GDM, that affect the pregnant subject and/or its offspring, include; preterm and caesarian delivery, birth injury to the mother or baby, shoulder dystocia, macrosomia, excessive offspring blood glucose concentration, excess weigh/adiposity and associated metabolic disorders e.g. type II diabetes, fatty liver disease and obesity immediately after birth and later in the life of the offspring, and an increased risk for the mother of having or developing type 2 diabetes immediately after birth and later in life. Accordingly, by treating or preventing GDM The combination of myo-inositol and probiotics and/or one or more of vitamin B2, B6, B12 and D may also be used to treat or prevent these conditions in these pregnant subjects or their offspring.

GDM is more prevalent amongst subjects who suffered with it in a previous pregnancy, if the subject is overweight or obese, if there is a family history of GDM or type II diabetes. If the subject is human and over 25 years of age, and if the subject is human and is black, Hispanic, American Indian or Asian.

Accordingly, myo-inositol and one or more probiotic, optionally further combined with one or more of vitamin B2, B6, B12 and D, may be particularly beneficial for treating and/or preventing GDM and preventing a condition associated therewith in one or more of these subsets of subjects and/or their offspring.

As would also be clear to the skilled person, the combination of myo-inositol and one or more probiotic, and any other optional ingredients disclosed herein, may be comprised in a composition, such as that disclosed herein, which may be employed in any method or use set out herein.

Further advantages and features of the present invention are apparent from the figure and non-limiting example. The present invention will now be described in further details by the way of the following examples.

### Examples

### Example 1

An example composition comprising a combination of myo-inositol and probiotics (Lactobacillus rhamnosus GG1 and Bifidobacterium lactis BB122) further combined within vitamin B2 and vitamins B6, B12 and D, is set out in table 1.

The composition in table I is for a nutritional supplement in a powder form, intended to be sprinkled on food.

**Table 1 : Composition of Example 1**

| Ingredient | Amount per daily dose |
|---|---|
| Myo-inositol | 4g |
| Vitamin D | 10 µg |
| Vitamin B6 | 2.6 mg |
| Vitamin B12 | 5.2 µg |
| Vitamin B2 | 1.8 mg |
| Zinc | 10 mg |
| β-carotene | 720 µg |
| Folic acid | 400 µg |
| Iron | 12 µg |
| Calcium | 150 µg |
| Iodine | 150 µg |
| Lactobacillus rhamnosus GG1) | 1x109 cfu |
| Bifidobacterium lactis BB122) | 1x109 cfu |

Strain deposited as CGMCC 1.3724
Strain deposited as CNCM I-3446

The composition may, for example, be provided as a kit of parts comprising in one sachet the probiotics as a powder and in a second sachet all other ingredients.

### Example 2

A milk powder drink for pregnant women to be reconstituted in water is provided consisting of 30g of milk powder per serving admixed with the ingredients listed in Table 1, in half of the amounts mentioned in Table 1. The composition is to be administered to a pregnant woman twice per day during at least the third trimester of pregnancy.

### Example 3

The nutritional deficiencies have been analysed in pregnant mothers in order to optimize the formulation of a composition for maternal administration to target more specifically the deficiencies of this specific population.

### Study design:

Two mother-infant cohorts were included in the analysis:

### A) SWS Cohort:

501 mother-infant pairs in the period of late pregnancy and taking part in the Southampton Women's Survey (SWS) were involved. Table 2 summarizes the characteristics of the SWS subjects analyzed.

**Table 2: Summary characteristics of the SWS subjects analyzed:**

| | Number | Percentage |
|---|---|---|
| Maternal Age (years) | | |
| <25 | 34 | 6.8% |
| 25-35 | 398 | 79.4% |
| ≥35 | 69 | 13.8% |

| Ethnicity | | |
|---|---|---|
| White Caucasian | 486 | 97.0% |
| Non-white Caucasian | 15 | 3.0% |

### B) GUSTO cohort:

500 mother-infant pairs in the period of late pregnancy, recruited from two major public maternity units in Singapore, namely National University Hospital (NUH) and KK Woman's Hospital (KKH) were involved. Mother's receiving chemotherapy, psychotropic drugs or who had type I diabetes mellitus were excluded. Table 3 summarizes the characteristics of the GUSTO subjects analyzed.

**Table 3: Summary characteristics of the GUSTO subjects analyzed:**

| | Number | Percentage |
|---|---|---|
| Maternal Age (years) | | |
| <25 | 85 | 17.0% |
| 25-35 | 307 | 61.4% |
| ≥35 | 108 | 21.6% |

| Ethnicity | | |
|---|---|---|
| Chinese | 252 | 50.4% |
| Malay | 162 | 32.4% |
| Indian | 86 | 17.2% |

The serum concentrations of different micronutrients were measured in pregnant women of the SWS cohort and the plasma concentrations of the same micronutrients were measured in pregnant women of the GUSTO cohort. For each cohort, the percentage of women showing deficiencies and/or insufficiencies of such nutrients was determined.

### Measurement of Vitamin D concentrations

Serum/plasma 25-hydroxyvitamin D [25(OH)D] concentrations were analyzed by radioimmunoassay (Diasorin, Stillwater, Minnesota, USA). This assay measures both 25(OH)D2 and 25(OH)D3. The assay met the requirements of the UK National Vitamin D External Quality Assurance Scheme, and intra- and interassay CVs were <10%.

Maternal vitamin D status was categorized according to 25-hydroxyvitamin D concentration as follows: <25 nmol/L "deficient," 25 to 50 nmol/L "insufficient," 50 to 70 nmol/L "borderline," and >70 nmol/L 'sufficient," as used by the UK National Diet and Nutrition Survey 2004.

Reference: Ruston D, Hoare J, Henderson L, et al. The National Diet and Nutrition Survey: Adults Aged 19-64 Years, Vol. 4: Nutritional Status (Anthropometry and Blood Analytes), Blood Pressure and Physical Activity. London: HMSO; 2004.

### Measurement of vitamin B12 concentration

Vitamin B12 was analysed by automated immunoassay, using 'Access Immunoassay systems' on the Beckman Coulter DXi 800, a continuous, random access analyser. The Access Vitamin B12 assay is a paramagnetic particle, chemiluminescent immunoassay for the quantitative determination of vitamin B12 levels in human serum and plasma (heparin) using the Access Immunoassay Systems (Beckman Dxl 800). The Access Vitamin B12 assay is a competitive binding immunoenzymatic assay which exhibits total imprecision of < 12% across the assay range. The lowest detectable level of Vitamin B12 distinguishable from zero with 95% confidence is 50 pg/mL (37 pmol/L).

The quality control data of the two machines used in the measurements are shown in the table below.

**Table 4: Quality control data (Machine 1 on the right, machine 2 on the left**

| | Level 1 | | Level 2 | | Level 3 | |
|---|---|---|---|---|---|---|
| Mean | 107.5 | 98.7 | 555.1 | 527.8 | 1093.4 | 1080.0 |
| SD | 9.65 | 12.2 | 42.4 | 34.9 | 107.1 | 126.4 |
| CV % | 9.65 | 12.35 | 6.29 | 8.04 | 9.79 | 11.7 |

The recommended cut-offs for diagnosing vitamin B12 deficiency and depletion are <148pmol/l (<200pg/ml) and <221 pmol/l (<300 pg/ml), respectively, in plasma or serum

A measured level of serum vitamin B12 <200 ng/L was defined as the limit of vitamin B12 deficient status.

Reference: Badiou S, Bariolet S, Laurens C, Aillaud N, Bargnoux AS, Mariano-Goulart D, Dupuy AM, Cristol JP. Comparison of isotopic and immunoenzymatic methods for folate and vitamin B12 determination. Clin Lab. 2010; 56:547-52. Simpson JL, Bailey LB, Pietrzik K, Shane B, Holzgreve W. Micronutrients and women of reproductive potential: required dietary intake and consequences of dietary deficiency or excess. Part I--Folate, Vitamin B12, Vitamin B6. J Matern Fetal Neonatal Med. 2010 Dec; 23(12):1323-43; Institute of Medicine and Committee (IOM). Dietary reference intake: folate, other B vitamins and choline. Washington, DC: National Academy Press; 199.8.

### Measurement of vitamin B6 (PLP) concentration

A reverse-phase high performance liquid chromatography (HPLC) method with post column derivatisation and fluorimetric detection was used to determine pyridoxal-5-phosphate (PLP). QC was achieved through internal procedures as there were no external quality schemes for the vitamin B6 HPLC method. QC material was produced by spiking human plasma with aqueous solutions of PLP. The final QC concentration was designed to match typical mid-range human samples and previously provided CDC 'mid bench' quality controls. The QC material was spiked so that the additional aqueous content represented only 0.02% of the total medium. Duplicate analysis of the QC material was performed with each analytical run. When the mean percentage recovery was outside of the range 95 to 105% of nominal the analytical results for that run were corrected accordingly. Good agreement between the obtained values for PLP in the quality control and the expected values indicates a high degree of accuracy for this method.

For a few of the samples repeated analysis was undertaken where the initial analysis yielded concentration values outside of the calibration ranges. An even smaller number had concentrations reported as less than the lowest calibration standard because, for the most part, it was not possible to re analyse these samples. This approach means that some re-analysed samples are reported as concentration values much lower than the bottom standard, while a few samples are represented as values less than the lowest measurable value. For statistical analysis we have assigned a value midway between 0 and the bottom of the calibration range to those (3 subjects) with values less than the bottom standard. One subject had no PLP peak, so her PLP concentration was set as "0".

Vitamin B6 status is usually assessed by plasma PLP levels. Exported from liver as a PLP-albumin complex, plasma PLP is considered a reflection of hepatic B6 levels and stores. Plasma PLP levels of <20 nmol/l is considered to reflect adverse vitamin status in the adult for assessing sufficiency. A level of serum/plasma PLP <20 nmol/L was thus was defined as the limit of vitamin B6 deficient status.

Reference: Rybak ME, Pfeiffer CM. Clinical analysis of vitamin B6: Determination of pyridoxal 50-phosphate and 4-pyridoxic acid in human serum by reversed-phase high-performance liquid chromatography with chlorite post column derivatization. Analytical Biochemistry 2004; 333:336-344. Lumeng L, Ryan MP, Li TK. Validation of the diagnostic value of plasma pyridoxal 50-phosphate measurements in vitamin B6 nutrition of the rat. J Nutr 1978;108:545-553; Li A, Lumeng L, Aronoff GR, Li T-K. Relationship between body store of vitamin B6 and plasma pyridoxal-P clearance: metabolic balance studies in humans. J Lab Clin Med 1985;106:491-497. Coburn SP, Lewis DL, Fink WJ, Mahuren JD, Schaltenbrand WE, Costill DL. Human vitamin B6 pools estimated through muscle biopsies. Am J Clin Nutr 1988; 48:291-294.

### Measurement of folate concentration

Serum/plasma folate concentration was analysed by automated immunoassay, using 'Access Immunoassay systems' on the Beckman Coulter DXi 800, a continuous, random access analyser.1 The Access Folate assay is a paramagnetic particle, chemiluminescent immunoassay for the quantitative determination of folic acid levels in human serum, plasma (heparin) or red blood cells using the Access Immunoassay Systems (Beckman Dxl 800). The Access Folate assay is a competitive binding receptor assay which exhibits total imprecision under 15% across the assay range. The lowest detectable level of folic acid distinguishable from zero with 95% confidence is 0.5 ng/mL (1.1 nmol/L).

The quality control data of the two machines used in the measurements are shown in the table below.

**Table 5: Quality control data (Machine 1 on the right, machine 2 on the left**

| | Level 1 | | Level 2 | | Level 3 | |
|---|---|---|---|---|---|---|
| Mean | 2.549 | 2.736 | 6.153 | 6.309 | 11.284 | 11.449 |
| SD | 0.213 | 0.243 | 0.376 | 0.350 | 0.505 | 0.431 |
| CV % | 8.35 | 8.9 | 6.11 | 5.55 | 4.47 | 3.76 |

For the analyses a level of folate <3 ng/ml was defined as the limit of folate deficient status.

Reference: 1. Badiou S, Bariolet S, Laurens C, Aillaud N, Bargnoux AS, Mariano-Goulart D, Dupuy AM, Cristol JP. Comparison of isotopic and immunoenzymatic methods for folate and vitamin B12 determination. Clin Lab. 2010;56:547-52.

### Measurement of iron concentration

Ferritin was measured by using a commercial ELISA kit (AssayMax Human Ferritin ELISA, AssayPro). Briefly, 50 µl of sample or standard or control was added on a pre-coated micro plate and was sandwiched by the immobilized antibody and biotinylated polyclonal antibody specific for ferritin, which is recognized by a streptavidin-peroxidase conjugate. All unbound material was then washed away and a peroxidase enzyme substrate was added. The color development was stopped and the intensity of the color was measured immediately on a micro plate reader at a wavelength of 450 nm.

A level of serum/plasma ferritin <15 ng/ml was considered to indicate iron depletion (deficiency).

Reference: Serum ferritin concentrations for the assessment of iron status and iron deficiency in populations as described by WHO in: Serum ferritin concentrations for the assessment of iron status and iron deficiency in populations. Vitamin and Mineral Nutrition Information System. Geneva, World Health Organization, 2011 (WHO/NMH/NHD/MNM/11.2).

### Measurement of zinc concentration

Serum/Plasma Zinc (Zn) concentrations were determined by inductively coupled plasma mass spectrometry (ICP-MS model Nexlon 300D, Perkin Elmer; Rotkreuz, Switzerland). Serum samples were defrosted and 50-100 µl serum was diluted to 5 ml in a plastic tube using 0.14 M HNO3 after addition of 100 µl of 1 mg/l In as internal standard. The ICP-MS was calibrated using standards containing 25, 50, 75, 100, µg/l of Zn and L-Cysteine hydrochloride monohydrate for matrix matching. Isotopes selected for analysis were 66Zn. The ICP-Ms was equipped with a glass cyclonic spray chamber and Meinhard nebulizer (300µl/min sample uptake). Interferences were removed using the collision mode with a He cell gas flow of 3.5 ml/min. Lyophilized human control serum (Seronorm Trace Element Serum, Level 1 and 2, Sero AS, Billingstad, Norway, Ref. No: 201405 and 203105) was analyzed for quality control together with the samples. Coefficient of variation of intermediate reproducibility CV(iR) of the method obtained with quality control samples Level 1 was 2.5% for Zn, respectively. Coefficient of variation of intermediate reproducibility CVn (iR) of the method obtained with quality control samples Level 2 was 3 2.0%. Recovery at both concentration levels was in the range of 94 - 107 %.

A level of serum/plasma Zn <700 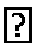 g/L was defined as the limit of zinc deficiency.

Reference: Hotz C, Peerson JM and Brown KH. Suggested lower cutoffs of serum zinc concentrations for assessing zinc status: reanalysis of the second National Health anNutrition Examination Survey data (1976-1980). Am J Clin Nutr. 2003;78:756-64.

### Results

The results of the analyses of serum/plasma samples indicated that during late pregnancy specific micronutrient deficiencies are observed.

Vitamin D insufficiency (<50nmol/L) was extremely prevalent during late pregnancy in women from the SWS cohort (39%) and from the GUSTO cohort (47%).

Vitamin B6 deficiency (<20 nmol/L) was extremely prevalent in women from the SWS cohort (70%) and common but not highly prevalent in the GUSTO cohort (16%).

Vitamin B12 deficiency (<200 ng/L) was extremely prevalent during late pregnancy in women from the SWS cohort (65%) and from the GUSTO cohort (62%).

Folate deficiency (<3 ng/ml) was rare in women from the SWS cohort (1%) and from the GUSTO cohort (1%).

Iron deficiency had low frequency in women from SWS cohort (4% with ferritin levels <15 ng/ml) and from the GUSTO cohort (7% with ferritin levels <30 nmol/L).

Zinc deficiency (<700 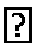 g/L) was prevalent in women from the SWS cohort (40%) and of low prevalence in women from the GUSTO cohort (4.8%).

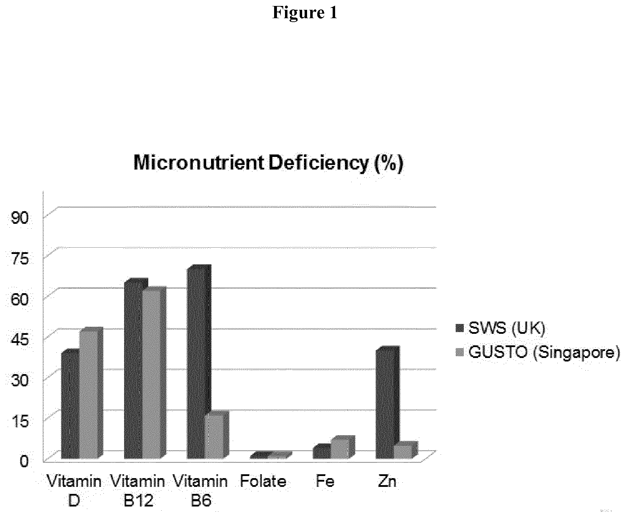

The results are summarized in Figure 1. These results therefore indicate that main nutrient deficiencies in pregnant women are vitamins B6, B12 and D, and in a smaller extent zinc. To the contrary, pregnant women are less often deficient in folate and iron.

### Example 4

In this study, the nutrient status of pregnant women was assessed in a representative sample of Chinese pregnant women by the way of a dietary intake survey and compared to the latest version of Chinese dietary recommended intakes (DRIs).

### Study design

### Study Subjects

600 pregnant women from 8 Chinese cities, including Beijing, Chengdu, Guangzhou, Lanzhou, Shanghai, Shenyang, Suzhou and Zhengzhou were contacted. 20% of them refused, while 480 women agreed to attend the survey. 1 out of 480 did not complete the questionnaire. Therefore, 479 questionnaires were deemed to be valid.

### Inclusion/Exclusion criteria

Pregnant women had to be generally healthy; had no evidence of chronic disease, no alcohol consumption (defined as more than two drinks per week), or smoking habits. Pregnant women with gestational diabetes, hypertension, infectious disease (tuberculosis, virus hepatitis and HIV infection), mental disease or recall deficit that could limit their ability to answer questions and receiving medical drugs during the period of the study were also excluded.

Once enrolled, participants completed several questionnaires including general information, anthropometry, living habits, nutrition supplements, food frequency questionnaire (FFQ) and one 24-hour dietary recall of the day before the survey with investigators in a one-to-one interview. Participants were instructed on how to complete the dietary records. Food models and measuring utensils were used to measure the serving sizes. Participants had to answer all questions on the form, otherwise the questionnaire was considered invalid.

### Statistical Analysis

Data entry was performed by two people at two separate computers using Epidata in order to guarantee the accuracy of the input.

### Nutrient intake assessment

Nutrient intakes were calculated by applying Chinese Food Composition Database (CFCD) 2002. A variety of nutrients were analyzed including Vitamin B2 (Riboflavin). Estimated average requirement (EAR) and tolerable upper intake level (UL) were used to determine the inadequacy and excess intake of nutrients.

References: Yang Yue-xin, WANG Guang-ya, PAN Xing-chang, et al. Chinese Food Composition Table2002[M]. Beijing: Pecking University Medical Press, 2002. 21-219. Chinese Nutrition Society. Chinese DRIs 2013[M]. Beijing. published date 2014.6.12.

### Food group intake assessment

Foods were categorized into eight major food groups according to the Balance Dietary Pagoda for Chinese Residents (published by the Chinese Nutrition Society). These categories are "grains, potatoes and legumes", "vegetables", "fruits", "meat and poultry", "fish and shrimp", "eggs", "milk and milk products", "soy and soy products and nuts", "oil", "salt", "water" and "beverages".

Daily consumption of "vegetables" was reported by 402 (96.2%) and only 15 (3.6%) of them reported to consume "vegetables" only weekly. "Fruits" were reported to be consumed by 416 (99.5%) women every day and the rest of them reported to consume "fruits" weekly.

### Results

The results of the nutrient intake analysis and of the adequacy of the intake against the Chinese DRIs are shown in tables 6 and 7, respectively.

**Table 6 : Average amounts of vitamin B2 intake by subject**

| | All subjects | First trimester | Second trimester | Third trimester |
|---|---|---|---|---|
| Vitamin B2 (mg) | 1.3±0.9 | 1.2±0.9 | 1.4±1.1 | 1.4±0.9 |

**Table 7 : Dietary reference intakes and assessment of subject intakes**

| | Dietary Reference Intakes (EAR) | % subjects with intake < EAR | % subjects with intake ≥ EAR |
|---|---|---|---|
| Vitamin B2 (mg) | 1.0 | 50.6 | 0.0 |

The results of the food group intake analysis and of the adequacy of the intake against the Chinese DRIs are shown in Table 8.

**Table 8 : Results of the food group intake analysis**

| Food groups | Lower DRI level (g/day) | Upper DRI level (g/day) | Average intake per subject | | Subjects with intake below DRI |
|---|---|---|---|---|---|
| | | | Mean±SD | N | % |
| Vegetables | 300 | 500 | 322.9±230.1 | 417 | 47.2 |
| Fruit | 200 | 400 | 362.6±310.2 | 416 | 27.6 |

The results of these analyses indicate that in China during pregnancy:
About half (50.6%) of the women have a lower that recommended intake of Vitamin B2 (<1 mg/day)
Nearly half (47.2%) of the women have lower than recommended intake of vegetables.

Nearly one third (27.6%) of the women have lower than recommended intake of fruits.

These results strongly support supplementation of maternal diet with vitamin B2 and nutrients found mainly in fruits and vegetables.

### Example 5

80 Females virgin Goto Kakizaki (GK) of 4-5 weeks old with close body weight, were purchased from Charles River (France and USA), caged singly in a temperature-controlled room (22±1°C) with a 12-h light/dark cycle, and maintained on a AIN-93G growing rodent diet (Research diets, USA, **Table 9**).

After one week on acclimation, GK rats were divided into 4 groups (n=20) after a randomization based on AUC glucose value and body weight, and received their corresponding treatment for 10 weeks as below:
1. Group Control: GK female rats ate *ad-libitum* a AIN-93G diet + a control gelatin (**Table10**).
2. Group Myo-inositol : GK female rats ate *ad-libitum* a AIN93-G diet supplemented with myo-inositol diet (1 g myo-inositol* for 100 g de diet) + a control gelatin (**Table10**).
3. Group Probiotic : GK female rats ate *ad-libitum* a AIN93-G + a gelatin containing 10⁹ LPR CFU and 10⁹ B.lactis CFU(**Table10**).
4. Group Mix : GK female ate *ad-libitum* a AIN93G diet supplemented with myo-inositol diet (1 g myo-inositol for 100g de diet) + a gelatin containing 10⁹ LPR CFU and 10⁹ B.lactis CFU (**Table10**).
*Kirsh Pharma

At 15 weeks of age, females were housed with Wistar males (Charles Rivers, France) until a vaginal plug was observed, indicating day 0.5 of gestation. Female rats remained on their allocated experimental diet throughout gestation. At 16.5 days of gestation an OGTT was performed on all females (n=20 per group) and at 19.5 days of gestation half of the animals (n=9-11 per group) were sacrificed after 6 hours fasting.

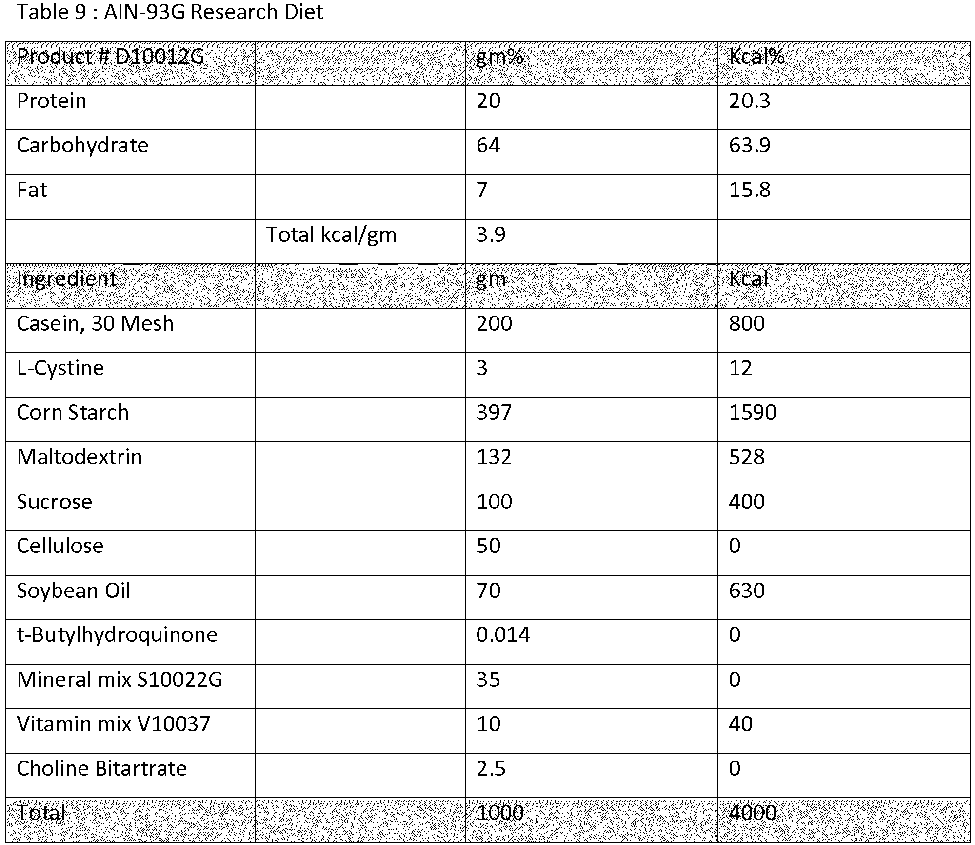

**Table 9.5 - Vitamin mix added to AIN-93G**

| **Ingredient** | **gm** | **Amount in 10gm** |
|---|---|---|
| Vitamin A, Acetate (500,000 IU/gm) | 0.8 | 4000 IU |
| Vitamin D3 (100,000 IU/gm) | 1 | 1,000 IU |
| Vitamin E Acetate (500 IU/gm) | 15 | 75 IU |
| Phylloquinone | 0.075 | 0.75 mg |
| Biotin, 1.0% | 2 | 0.2 mg |
| Cyanocobalamin, 0.1% | 2.5 | 25 µg |
| Folic Acid | 0.2 | 2 mg |
| Nicotinic Acid | 3 | 30 mg |
| Calcium Pantothenate | 1.6 | 16 mg |
| Pyridoxine---HCl | 0.7 | 7 mg |
| Riboflavin | 0.6 | 6 mg |
| Thiamin HCl | 0.6 | 6 mg |
| Sucrose | 971.925 | |
| **TOTAL** | **1000** | |

**Table 10 : Composition of the experimental Gelatin**

| | Treatment | Control |
|---|---|---|
| Gelatin (g) | 12 | 12 |
| Sacharine (mg) | 0.06 | 0.06 |
| Probiotic (g)* | 7.7 | 0 |
| Maltodextrin mix (g) | 0 | 6.7 |
| TS + Water (ml) | 80.2 | 81.2 |
| Total (ml) | 99.96 | 99.96 |

| | | |
|---|---|---|
| *Premix containing 2.53 E+10 cfu/g Lactobacillts rhamnosus (NCC4007) and 1.58E+10 cfu/g of Bifido Lactis (NCC2818). | | |

### Measurements and analysis

### Physiological measurements and sample collections:

- An oral glucose tolerance test (OGTT) was performed after 6 hours of day deprivation (from 7.30 am to 13.30 pm) in all animals at age of 5 weeks and at 16.5 days of gestation. During OGTT two baseline blood samples were taken from the tail vein, with at least 10 minutes interval between sampling (time -10 and 0), followed by an oral gavage of glucose solution (20% wt/v) at dose of 1.5 g/Kg body weight. Then further blood samples were collected from tail incision at 15, 30, 45, 60, 90 and 120 min after glucose administration. All blood samples were analysed directly for blood glucose level, using Contour Next glucometers (Bayer AG, Zurich, Switzerland).
- The body weight and food intake of the animals were measured 2 times / week throughout the study.
- Body composition (body fat, lean mass and body water content) was measured before and after 10 weeks of treatment by using nuclear magnetic resonance (EchoMRI TM 2004, Echo Medical Systems, Houston, USA). From these data collected it was possible to calculate the delta %fat and the fat gain / weight gain ratio as following :
- The delta %fat: (% fat at the end of the treatment - % fat before the start of the treatment)
- Fat gain / weight gain : (fat (g) at the end of the treatment period - fat(g) before the start of the treatment) / (body weight (g) at the end of the treatment period - body weight (g) before the start of the treatment)
- lean gain / weight gain : (lean (g) at the end of the treatment period - lean (g) before the start of the treatment) / (body weight (g) at the end of the treatment period - body weight (g) before the start of the treatment)
- Fat gain : fat (g) at the end of the treatment period - fat(g) before the start of the treatment
- lean gain : lean (g) at the end of the treatment period - lean (g) before the start of the treatment
- % fat mass : fat mass (g) / body weight^{∗}100
- % lean mass : lean mass (g) / body weight^{∗}100

Sacrifice: dams were decapitated after 6 hours of day deprivation (from 7.30 am to 13.30 pm) and organ collected by dissection, weighted and immediately frozen in liquid nitrogen. They were kept at -80°C until analysis.
- Insulin pancreatic measurement: Pancreas was weighted during the sacrifice and preserved in an acid-ethanol-H2O solution until insulin extraction For determination of pancreatic insulin, pancreas was homogenized with an acid-ethanol (solution v/v: 75% ethanol, 1.5% of 37% HCI and 23.5% distilled water) and incubated at -20°C overnight. The insulin content in the supernatant was measured by an ELISA method using kits from Crystal Chem. Inc (IL, USA).

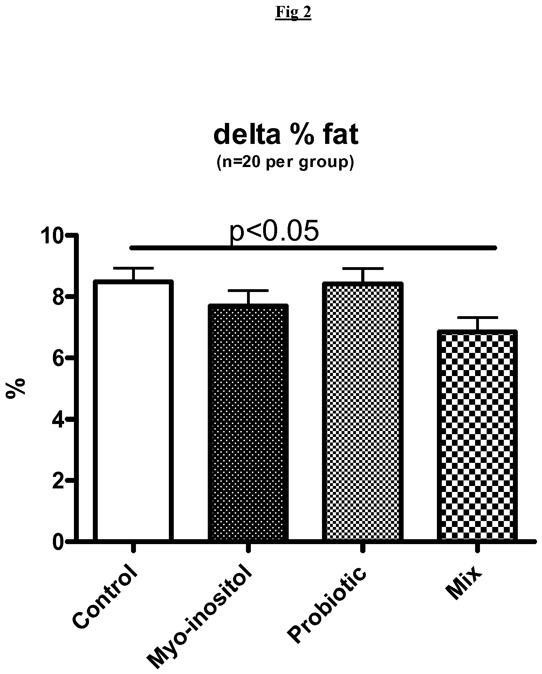

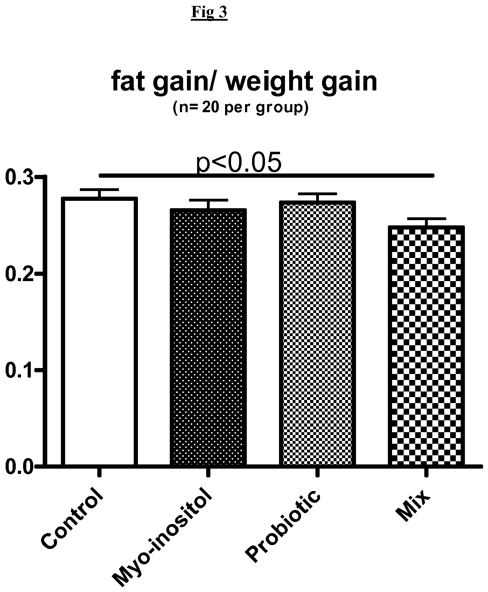

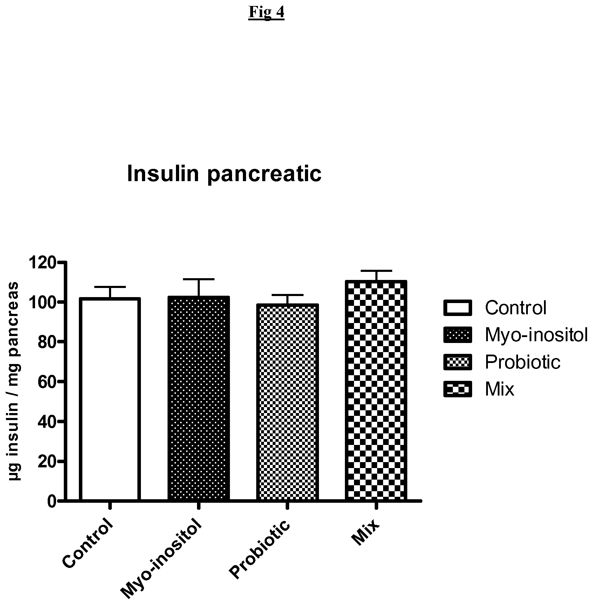

Results: The mean value for each measurement is shown in table 11. Results for delta % fat, fat gain/ weight gain, µg insulin pancreas/ g pancreas can also be seen in Figures 2 to 4.

**Table 11**

| | Control | Myo-inositol | Probiotics | Mix (myo-inositol+probiotics) |
|---|---|---|---|---|
| % fat mass | 19.5 | 18.7 | 19.3 | 18.1 |
| % lean mass | 66.4 | 67.8 | 67.3 | 68.3 |
| Delta % fat | 8.8 | 7.7 | 8.4 | 6.8 |
| Fat gain/ weight gain | 0.28 | 0.27 | 0.27 | 0.24 |
| Lean gain/ weight gain | 0.57 | 0.58 | 0.58 | 0.60 |
| % fat gain | 27.6 | 26.6 | 27.3 | 24.7 |
| % lean gain | 57.4 | 58.3 | 58.5 | 60.3 |
| Body weight gain | 107 | 100 | 105 | 102 |
| Fat gain (g) | 29.8 | 26.8 | 28.8 | 25.3 |
| Lean gain (g) | 61.7 | 58.5 | 61.3 | 61.5 |
| ug insulin pancreas/ g pancreas | 101 | 101 | 98.5 | 110.3 |
| AUC insulin increase | 2 | 5 | -2 | 13 |
| FGIR | 3.44 | 3.42 | 3.24 | 4.21 |
| HOMA1-IR | 10.42 | 9.29 | 9.76 | 9.07 |

### Example 6

80 Females virgin Goto Kakizaki (GK) rats of 4-5 weeks old with close body weight, were purchased from Charles River (France and USA), caged singly in a temperature-controlled room (22±1°C) with a 12-h light/dark cycle, and maintained on a AIN-93G growing rodent diet (Research diets, USA, **Table 9**).

After one week on acclimation, GK rats were divided into 4 groups (n=20) after a randomization based on AUC glucose value and body weight, and received their corresponding treatment for 10 weeks as below:
- Group Control: GK female rats ate *ad-libitum* a AIN-93G diet + a control gelatin (**Table10**).
- Group Myo-inosito: GK female rats ate *ad-libitum* a AIN93-G diet supplemented with myo-inositol diet (1 g myo-inositol* for 100 g de diet) + a control gelatin (**Table10**).
- Group Probiotic: GK female rats ate *ad-libitum* a AIN93-G + a gelatin containing 10⁹ LPR CFU and10⁹ B.lactis CFU(**Table10**).
- Group Mix : GK female ate *ad-libitum* a AIN93G diet supplemented with myo-inositol diet (1 g myo-inositol for 100g de diet) + a gelatin containing 10⁹ LPR CFU and 10⁹ B.lactis CFU (**Table10**).
*Kirsh Pharma

At 15 weeks of age, females were housed with Wistar males (Charles Rivers, France) until a vaginal plug was observed, indicating day 0.5 of gestation. Female rats remained on their allocated experimental diet throughout gestation. At birth, only dams and their pups bearing at least 8 pups with minimum 3-4 males per litter, were included in the study. The number of pups in each litter was limited to 8, with preference to male pups. All dams were fed ad-libitum with a AIN-93G growing rodent diet (Research diets, USA, **Table 1**) during the lactation period. The pups remained with their mother until age of 21 days. During this period, they were allowed to suckle ad-libitum from dams. Then, they were housed two per cages and were fed an AIN93-G from d22 to d53. Body weight of the offspring was measured once per week, body composition was assessed at 21 days of age and all animals were sacrified at 100 days old.

### Measurements and analysis

### Physiological measurements and sample collections:

- The body weight of the offspring was measured 1-2 times / week throughout the study.
- Lean body mass was measured at 21 days of age by using nuclear magnetic resonance (EchoMRI TM 2004, Echo Medical Systems, Houston, USA). From these data collected it was possible to calculate the %lean mass as following :
   - % lean mass : lean mass (g) / body weight

Sacrifice: offspring were sacrificed under under anesthesia (isofluarane) after 6 hours of day deprivation (from 7.30 am to 13.30 pm). Blood was collected in EDTA tubes and plasma FFA was measured by Cobas. (Cobas C111, Roche).
- Insulin pancreatic measurement: Pancreas was weighted during the sacrifice and preserved in an acid-ethanol-H2O solution until insulin extraction. For determination of pancreatic insulin, pancreas was homogenized with an acid-ethanol (solution v/v: 75% ethanol, 1.5% of 37% HCI and 23.5% distilled water) and incubated at -20°C overnight. The insulin content in the supernatant was measured by an ELISA method using kits from Crystal Chem. Inc (IL, USA).
Results: The mean value for each measurement is shown in table 12

**Table 12**

| | Control | Myo-inositol | Probiotics | Mix (myo-inositol+probiotics) |
|---|---|---|---|---|
| % lean mass at 21 days old | 82.8 | 81.8 | 82.1 | 83.2 |
| lean mass in g at 21 days old | 36.2 | 39.3 | 38.2 | 34.6 |
| Body weight in g at 21 days old | 44.3 | 48.1 | 46.4 | 43.9 |
| µg insulin in total pancreas | 84 | 89 | 90 | 107 |
| µg insulin pancreas/ g pancreas | 68 | 76 | 75 | 89 |
| FFA plasmatic (mmol/l) | 0.43 | 0.41 | 0.43 | 0.40 |

### Example 7

Measurement of how supplementation of myoinositol and probiotics affects transcription of SLC5A11 in rat gastrocnemius during pregnancy.

Gene expression analysis was performed on muscle (gastrocnemius) tissue samples of 10 dams of each group described in example 6. Rats were sacrificed under anesthesia (isofluarane) at 19.5 days of gestation after 6 hours fasting. This analysis was done using the GeneChip® Rat Genome 230 2.0 Array that provides comprehensive coverage of the transcribed rat genome using more than 31,000 probe sets, analyzing over 30,000 transcripts and variants from over 28,000 well-substantiated rat genes.

The steps performed for each sample are the following:

### 1) Sample preparation for microarrays analysis.

First, the tissue was pulverized using a Covaris cryoPREP™ CP02 (Covaris Ltd), and then 10-20 mg of powder were transfered to tubes frozen in liquid nitrogen to avoid thawing the samples. Then, lysis and extraction was done using the FastPrep and Agencourt RNAdvance Tissue Kit protocol (vers. Jan 2015). Once extracted RNA was quantified using the Quant-it Quant-iT™ RiboGreen® RNA Reagent and Kit, and the quality control was performed using the Standard Sensitivity RNA analysis Kit and the Fragment Analyzer (Advanced Analytical Technologies, Inc). All samples with a RQN>7.5 were eligible for cRNA synthesis. cRNA synthesis was performed using the Illumina® TotalPrep™-96 RNA Amplification Kit, starting with 300ng of Total RNA in 9µL Nuclease-free water, and performing a modification to the commercial protocol to use the Agencourt RNAclean XP kit for the purification steps instead of using the two purifications steps of the Illumina TotalPrep-96 kit. cRNA was then quantified and the Quality Control performed using the Quant-it Quant-iT™ RiboGreen® RNA Reagent and Kit and the Standard Sensitivity RNA analysis Kit, respectively. The Fragment Analyzer (Advanced Analytical Technologies, Inc) was used and the expected average size should be of 1580nt or greater. Finally, the cRNA was fragmented using a 5X Array Fragmentation Buffer (Affymetrix Kit IVT) and samples were incubated for 35 min at 94°C.Finally, the hybridization was prepared, first a mastermix was generated containing per sample 3.7µLof Control Oligo B2 (3 nM), 11µL of 20X Hybridization Controls (must be heated 5 min at 65°C before), 110µLof 2X Hybridization Mix, 22µL of DMSO and 43.9µl of Nuclease-free Water. For each sample 29.4µL fragmented cRNA + 190.6µL hybridization mix were added in a 250 µL PCR plate, ad these hybridization cocktails were kept at -20°C until use or -80°C if use is not in the same week. 2) Microarrays Hybridization.

For this step the GeneChip® Hybridization Wash and Stain Kit (part nr: 900720, Affymetrix, Inc.) was used. First, the Hybridization Cocktail was heated to 99 °C for 5 minutes and 45°C for another 5 min in a PCR machine. Meanwhile, per array (Rat Genome 230 2.0 Array,2pk GeneChip, part nr: 900505, Affymetrix, Inc.) 200µL of Pre-Hybridization Mix were used to fill the probe array and then proceed to incubate at 45 °C in the oven (Affymetrix Hybridization Oven 645, Affymetrix, Inc) for 10 minutes with rotation. Then, the array was removed from the hybridization oven and the pre-hyb solution removed to then be refilled t with 200µL of Hybridization Cocktail. Finally, the probe array was placed in the hybridization oven, set to 45 °C and it was hybridized for 16 hours.

### 3) Microarrays Staining and scanning

For this the GeneChip® Expression Wash, Stain and Scan protocol was strictly followed as described by the manufacturer. Arrays were processed using the Affymetrix Fluidic Station 450, the Affymetrix GeneChip Autoloader and the Affymetrix GeneChip Scanner. The gene expression levels data were extracted and analyzes using a workflow from a dedicated commercial software (Partek®)

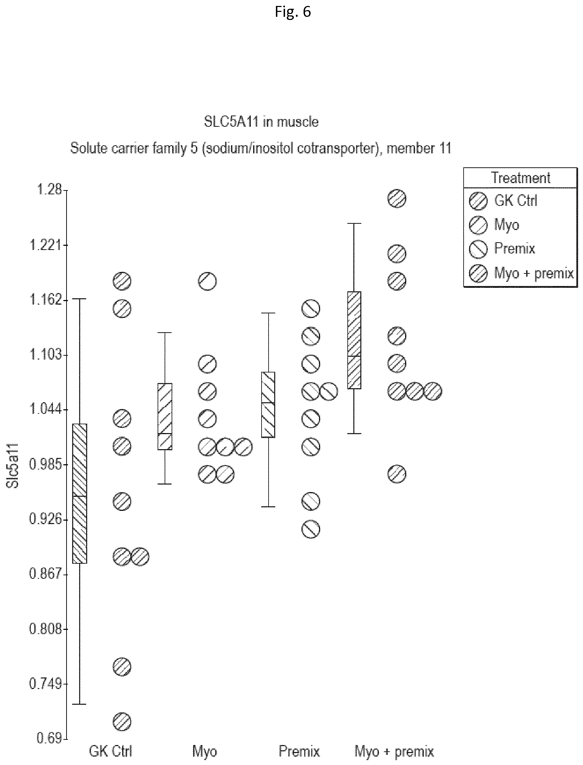

Results are shown in Figures 6

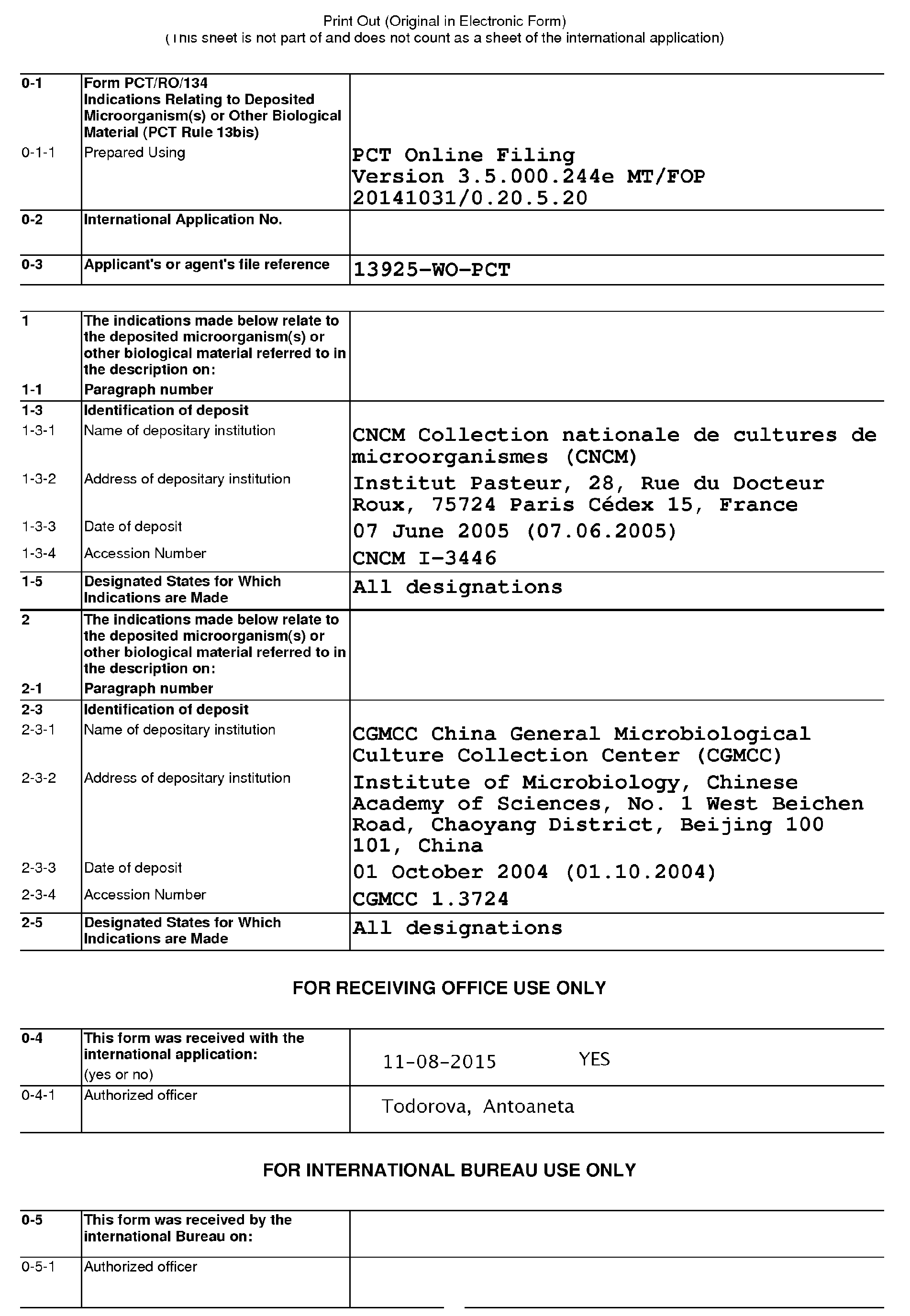

## Claims

1. A composition for maternal administration comprising myo-inositol and probiotics for use to minimise excessive fat accretion in a pregnant subject or a subject desiring to become pregnant, and/or to treat or prevent gestational diabetes mellitus and a condition associated therewith in a pregnant subject and/or its offspring, wherein said composition is administered to said subject before and/or during pregnancy and/or during lactation and, wherein the probiotics comprise a combination of Lactobacillus and Bifidobacterium, and wherein preferably the Lactobacillus is the Lactobacillus rhamnosus GG strain available under the deposit number CGMCC 1.3724, and/or the Bifidobacterium is the Bifidobacterium lactis BB12 strain deposited as CNCM 1-3446.

2. A composition for use according to claim 1 wherein said composition further comprises at least one vitamin selected from vitamin B2, vitamin B6, vitamin B12, vitamin D and mixtures thereof.

3. A composition for use according to claim 2 wherein if said composition comprises vitamin B2, vitamin B2 is present in the composition in an amount of from 0.14 to 14 mg per daily dose, if said composition comprises vitamin B6, vitamin B6 is present in an amount of from 0.19 to 19 mg per daily dose, if said composition comprises vitamin B12, vitamin B12 is present in an amount of from 0.26 to 26 µg per daily dose; and if said composition comprises vitamin D, vitamin D is present in an amount of from 1.5 to 100 µg per daily dose wherein, daily dose refers to a daily quantity that is administered to a subject before and/or during pregnancy and/or during lactation.

4. A composition for use according to any one of the preceding claims, **characterized in that** it further comprises zinc.

5. A composition for use according to any one of the preceding claims comprising myo-inositol, vitamin B2, vitamin B6, vitamin B12, vitamin D, Bifidobacterium lactis BB12 CNCMI-3446 and Lactobacillus rhamnosus GG CGMCC 1.3724, and preferably from 0.2 to 5 g of myo-inositol, from 0.14 to 14 mg of vitamin B2, from 0.19 to 19 mg of vitamin B6, from 0.26 to 26 µg of vitamin B12, from 1.5 to 100 µg of vitamin D, from 10⁵ to 10¹² cfu of Bifidobacterium lactis BB12 CNCMI-3446 and a from 10⁵ to 10¹² cfu of Lactobacillus rhamnosus GG CGMCC 1.3724, all amounts being defined by daily dose wherein, daily dose refers to a daily quantity that is administered to a subject before and/or during pregnancy and/or during lactation.

6. A composition for use according to any one of the preceding claims wherein it is in the form of a powdered nutritional composition to be reconstituted in milk or water, a food product, a drink, a nutritional supplement or a nutraceutical.

7. A composition for use according to any one of claim 1 to 6 wherein the subject is a mammal selected from the group consisting of a cat, a dog, a human, and wherein said composition is preferably administered enterally, more preferably orally, and preferably is a powdered nutritional composition, a food product, a drink, a nutritional supplement such as a powdered nutritional supplement, or a nutraceutical.

8. A composition for use according to anyone of claims 1 to 7, wherein the condition associated with gestational diabetes mellitus is preterm and caesarean delivery, birth injury to the mother or baby, shoulder dystocia, macrosomia, excessive offspring blood glucose concentration, excess weigh/adiposity and associated metabolic disorders e.g. type II diabetes, fatty liver disease and obesity immediately after birth and later in the life of the offspring, and an increased risk for the mother of having or developing type 2 diabetes immediately after birth and later in life.

9. A combination of myo-inositol and one or more probiotic for use to minimise excessive fat accretion in a pregnant subject or a subject desiring to become pregnant, and/or to treat or prevent gestational diabetes mellitus and a condition associated therewith in a pregnant subject and/or its offspring, wherein said combination of myo-inositol and one or more probiotic is administered to said subject before and/or during pregnancy and/or during lactation, wherein the probiotic is selected from the group consisting of; Lactobacillus, bifidobacterium, and combinations thereof, and more preferably selected from the group consisting of lactobacillus rhamnosus GG (CGMCC 1.3724), bifidobacterium lactus BB-12 (CNCM 1-3446), and a combination thereof, and wherein said myo-inositol and one or more probiotic are administered simultaneously, separately or sequentially.

10. A combination of myo-inositol and one or more probiotic for use according to claim 9 wherein said combination of myo-inositol and one or more probiotic is administered in combination with one or more of vitamin B2, B6, B12 and D, and wherein said myo-inositol and/or one or more probiotic is administrated separately or sequential to one or more of vitamin B2, B6, B12 and D, and wherein the condition associated with gestational diabetes mellitus is preferably preterm and caesarian delivery, birth injury to the mother or baby, shoulder dystocia, macrosomia, excessive offspring blood glucose concentration, excess weigh/adiposity and associated metabolic disorders e.g. type II diabetes, fatty liver disease and obesity immediately after birth and later in the life of the offspring, and an increased risk for the mother of having or developing type 2 diabetes immediately after birth and later in life.

## Patentansprüche

1. Zusammensetzung zur maternalen Verabreichung, umfassend Myoinosit und Probiotika zum Gebrauch zum Minimieren übermäßiger Fettansammlung bei einem schwangeren Subjekt oder einem Subjekt, das schwanger werden soll, und/oder zum Behandeln oder Vorbeugen von Gestationsdiabetes mellitus und einem damit verbundenen Zustand bei einem schwangeren Subjekt und/oder dessen Nachwuchs, wobei die Zusammensetzung dem Subjekt vor und/oder während der Gestation und/oder während der Laktation verabreicht wird und wobei die Probiotika eine Kombination von Lactobacillus und Bifidobacterium umfassen und wobei vorzugsweise der Lactobacillus der Stamm Lactobacillus rhamnosus GG ist, der unter der Depotnummer CGMCC 1.3724 erhältlich ist, und/oder das Bifidobacterium der Stamm Bifidobacterium lactis BB12 ist, der als CNCM 1-3446 hinterlegt ist.

2. Zusammensetzung zum Gebrauch nach Anspruch 1, wobei die Zusammensetzung ferner mindestens ein Vitamin umfasst, das aus Vitamin B2, Vitamin B6, Vitamin B12, Vitamin D und Mischungen davon ausgewählt ist.

3. Zusammensetzung zum Gebrauch nach Anspruch 2, wobei, wenn die Zusammensetzung Vitamin B2 umfasst, Vitamin B2 in der Zusammensetzung in einer Menge von 0,14 bis 14 mg pro Tagesdosis vorhanden ist, wenn die Zusammensetzung Vitamin B6 umfasst, Vitamin B6 in einer Menge von 0,19 bis 19 mg pro Tagesdosis vorhanden ist, wenn die Zusammensetzung Vitamin B12 umfasst, Vitamin B12 in einer Menge von 0,26 bis 26 µg pro Tagesdosis vorhanden ist; und wenn die Zusammensetzung Vitamin D umfasst, Vitamin D in einer Menge von 1,5 bis 100 µg pro Tagesdosis vorhanden ist wobei sich Tagesdosis auf eine tägliche Menge bezieht, die einem Subjekt vor und/oder während der Gestation und/oder während der Laktation verabreicht wird.

4. Zusammensetzung zum Gebrauch nach einem der vorstehenden Ansprüche, **dadurch gekennzeichnet, dass** sie ferner Zink umfasst.

5. Zusammensetzung zum Gebrauch nach einem der vorstehenden Ansprüche, umfassend Myoinosit, Vitamin B2, Vitamin B6, Vitamin B12, Vitamin D, Bifidobacterium lactis BB12 CNCMI-3446 und Lactobacillus rhamnosus GG CGMCC 1.3724 und vorzugsweise 0,2 bis 5 g Myoinosit, 0,14 bis 14 mg Vitamin B2, 0,19 bis 19 mg Vitamin B6, 0,26 bis 26 µg Vitamin B12, 1,5 bis 100 µg Vitamin D, 10⁵ bis 10¹² KBE Bifidobacterium lactis BB12 CNCMI-3446 und 10⁵ bis 10¹² KBE Lactobacillus rhamnosus GG CGMCC 1.3724, wobei alle Mengen durch die Tagesdosis definiert werden, wobei sich Tagesdosis auf eine tägliche Menge bezieht, die einem Subjekt vor und/oder während der Gestation und/oder während der Laktation verabreicht wird.

6. Zusammensetzung zum Gebrauch nach einem der vorstehenden Ansprüche, wobei sie in der Form einer pulverförmigen Nährstoffzusammensetzung vorliegt, die in Milch oder Wasser, einem Nahrungsmittelprodukt, einem Getränk, einem Nahrungsergänzungsmittel oder einem Nutrazeutikum zu rekonstituieren ist.

7. Zusammensetzung zum Gebrauch nach einem von Anspruch 1 bis 6, wobei das Subjekt ein Säugetier ist, ausgewählt aus der Gruppe bestehend aus einer Katze, einem Hund, einem Menschen, und wobei die Zusammensetzung vorzugsweise enteral, mehr bevorzugt oral verabreicht wird und vorzugsweise eine pulverförmige Nährstoffzusammensetzung, ein Nahrungsmittelprodukt, ein Getränk, ein Nahrungsergänzungsmittel, wie ein pulverförmiges Nahrungsergänzungsmittel, oder ein Nutrazeutikum ist.

8. Zusammensetzung zum Gebrauch nach einem der Ansprüche 1 bis 7, wobei die Bedingung im Zusammenhang mit Gestationsdiabetes mellitus vorzeitige Entbindung oder Kaiserschnittentbindung, Geburtsverletzung bei Mutter oder Baby, Schulterdystokie, Makrosomie, erhöhter Blutzuckerspiegel beim Nachwuchs, Übergewicht/Adipositas und damit verbundene Stoffwechselstörungen, z. B. Typ-2-Diabetes, Fettlebererkrankung und Fettleibigkeit des Nachwuchses unmittelbar nach der Geburt und später im Leben und ein erhöhtes Risiko für die Mutter, Typ-2-Diabetes unmittelbar nach der Geburt und später im Leben zu haben oder zu entwickeln, ist.

9. Kombination von Myoinosit und einem oder mehreren Probiotika zum Gebrauch zum Minimieren übermäßiger Fettansammlung bei einem schwangeren Subjekt oder einem Subjekt, das schwanger werden soll, und/oder zum Behandeln oder Vorbeugen von Gestationsdiabetes mellitus und einem damit verbundenen Zustand bei einem schwangeren Subjekt und/oder dessen Nachwuchs, wobei die Kombination von Myoinosit und einem oder mehreren Probiotika dem Subjekt vor und/oder während der Gestation und/oder während der Laktation verabreicht wird, wobei das Probiotikum ausgewählt sind aus der Gruppe, bestehend aus Lactobacillus, Bifidobacterium und Kombinationen davon, und mehr bevorzugt ausgewählt ist aus der Gruppe bestehend aus Lactobacillus rhamnosus GG (CGMCC 1.3724), Bifidobacterium lactus BB-12 (CNCM 1-3446) und einer Kombination davon, und wobei das Myoinosit und das eine oder die mehreren Probiotika gleichzeitig, separat oder nacheinander verabreicht werden.

10. Kombination von Myoinosit und einem oder mehreren Probiotika zum Gebrauch nach Anspruch 9, wobei die Kombination von Myoinosit und einem oder mehreren Probiotika in Kombination mit einem oder mehreren von Vitamin B2, B6, B12 und D verabreicht wird und wobei das Myoinosit und/oder das eine oder die mehreren Probiotika separat von oder sequenziell zu einem oder mehreren von Vitamin B2, B6, B12 und D verabreicht werden und wobei der Zustand im Zusammenhang mit Gestationsdiabetes mellitus vorzugsweise vorzeitige Entbindung oder Kaiserschnittentbindung, Geburtsverletzung für Mutter oder Baby, Schulterdystokie, Makrosomie, erhöhter Blutzuckerspiegel beim Nachwuchs, Übergewicht/Adipositas und damit verbundene Stoffwechselstörungen, z. B. Typ-2-Diabetes, Fettlebererkrankung und Fettleibigkeit des Nachwuchses unmittelbar nach der Geburt und später im Leben und ein erhöhtes Risiko für die Mutter, Typ-2-Diabetes unmittelbar nach der Geburt und später im Leben zu haben oder zu entwickeln, ist.

## Revendications

1. Composition pour administration maternelle comprenant du myo-inositol et des probiotiques, utilisable pour limiter l'accumulation excessive de graisse chez un sujet gestant ou un sujet souhaitant une grossesse, et/ou pour traiter ou prévenir le diabète sucré gestationnel et une pathologie y étant associée chez un sujet gestant et/ou sa progéniture, dans laquelle ladite composition est administrée audit sujet avant et/ou pendant la gestation et/ou pendant l'allaitement et, dans laquelle les probiotiques comprennent une combinaison de Lactobacillus et de Bifidobacterium, et dans laquelle préférablement le Lactobacillus est la souche de Lactobacillus rhamnosus GG disponible sous le numéro de dépôt CGMCC 1.3724, et/ou le Bifidobacterium est la souche de Bifidobacterium lactis BB12 déposée en tant que CNCM 1-3446.

2. Composition pour utilisation selon la revendication 1, dans laquelle ladite composition comprend en outre au moins une vitamine choisie parmi la vitamine B2, la vitamine B6, la vitamine B12, la vitamine D et des mélanges de celles-ci.

3. Composition pour utilisation selon la revendication 2, dans laquelle si ladite composition comprend de la vitamine B2, la vitamine B2 est présente dans la composition en une quantité de 0,14 à 14 mg par dose quotidienne, si ladite composition comprend de la vitamine B6, la vitamine B6 est présente en une quantité de 0,19 à 19 mg par dose quotidienne, si ladite composition comprend de la vitamine B12, la vitamine B12 est présente en une quantité de 0,26 à 26 µg par dose quotidienne ; et si ladite composition comprend de la vitamine D, la vitamine D est présente en une quantité de 1,5 à 100 µg par dose quotidienne, la dose quotidienne désigne une quantité quotidienne qui est administrée à un sujet avant et/ou pendant la gestation et/ou pendant l'allaitement.

4. Composition pour utilisation selon l'une quelconque des revendications précédentes, **caractérisée en ce qu'**elle comprend également du zinc.

5. Composition pour utilisation selon l'une quelconque des revendications précédentes comprenant du myo-inositol, de la vitamine B2, de la vitamine B6, de la vitamine B12, de la vitamine D, du Bifidobacterium lactis BB12 CNCMI-3446 et du Lactobacillus rhamnosus GG CGMCC 1.3724, et de préférence de 0,2 à 5 g de myo-inositol, de 0,14 à 14 mg de vitamine B2, de 0,19 à 19 mg de vitamine B6, de 0,26 à 26 µg de vitamine B12, de 1,5 à 100 µg de vitamine D, de 10⁵ à 10¹² ufc de Bifidobacterium lactis BB12 CNCMI-3446 et de 10⁵ à 10¹² ufc de Lactobacillus rhamnosus GG CGCCM 1.3724, toutes les quantités étant définies par dose quotidienne, dans laquelle la dose quotidienne désigne une quantité quotidienne qui est administrée à un sujet avant et/ou pendant la gestation et/ou pendant l'allaitement.

6. Composition pour utilisation selon l'une quelconque des revendications précédentes, dans laquelle elle est sous la forme d'une composition nutritionnelle en poudre à reconstituer dans du lait ou de l'eau, un produit alimentaire, une boisson, un complément nutritionnel ou un nutraceutique.

7. Composition pour utilisation selon l'une quelconque des revendications 1 à 6, dans laquelle le sujet est un mammifère choisi parmi le groupe constitué par un chat, un chien, un humain, et dans laquelle ladite composition est de préférence administrée par voie entérale, plus préférablement par voie orale, et est de préférence une composition nutritionnelle en poudre, un produit alimentaire, une boisson, un complément alimentaire tel qu'un complément alimentaire en poudre, ou un nutraceutique.

8. Composition pour utilisation selon l'une quelconque des revendications 1 à 7, dans laquelle la pathologie associée au diabète sucré gestationnel est un accouchement prématuré et par césarienne, une lésion à la naissance sur la mère ou sur bébé, une dystocie des épaules, une macrosomie, une glycémie excessive de la progéniture, une surcharge pondérale/adiposité et des troubles métaboliques associés, p. ex. un diabète de type II, une stéatose hépatique et une obésité immédiatement après la naissance et plus tard dans la vie de la progéniture, et un risque accru pour la mère de présenter ou de développer un diabète de type 2 immédiatement après la naissance et plus tard dans la vie.

9. Combinaison de myo-inositol et d'un ou plusieurs probiotiques, utilisable pour minimiser une accumulation excessive de graisse chez un sujet gestant ou un sujet souhaitant une grossesse, et/ou pour traiter ou prévenir le diabète sucré gestationnel et une pathologie associée chez un sujet gestant et/ou sa progéniture, dans laquelle ladite combinaison de myo-inositol et d'un ou plusieurs probiotiques est administrée audit sujet avant et/ou pendant la gestation et/ou pendant l'allaitement, dans laquelle le probiotique est choisi parmi le groupe constitué de ; Lactobacillus, Bifidobacterium, et leurs combinaisons, et plus préférablement choisi parmi le groupe constitué de Lactobacillus rhamnosus GG (CGMCC 1.3724), Bifidobacterium lactus BB-12 (CNCM 1-3446), et une combinaison de ceux-ci, et dans laquelle ledit myo-inositol et un ou plusieurs probiotiques sont administrés simultanément, séparément ou séquentiellement.

10. Combinaison de myo-inositol et d'un ou plusieurs probiotiques pour utilisation selon la revendication 9, dans laquelle ladite combinaison de myo-inositol et d'un ou plusieurs probiotiques est administrée en combinaison avec une ou plusieurs parmi la vitamine B2, B6, B12 et D, et dans laquelle ledit myo-inositol et/ou un ou plusieurs probiotiques sont administrés séparément ou séquentiellement par rapport à une ou plusieurs parmi la vitamine B2, B6, B12 et D, et dans laquelle la pathologie associée au diabète sucré gestationnel est de préférence un accouchement avant terme et par césarienne, une lésion à la naissance sur la mère ou sur le bébé, une dystocie des épaules, une macrosomie, une glycémie excessive de la progéniture, une surcharge pondérale/adiposité et des troubles métaboliques associés p. ex. un diabète de type II, une stéatose hépatique et une obésité immédiatement après la naissance et plus tard dans la vie de la progéniture, et un risque accru pour la mère de présenter ou de développer un diabète de type 2 immédiatement après l'accouchement et plus tard dans la vie.
